(19) Europäisches Patentamt
European Patent Office
Office européen des brevets

(11) Numéro de publication : **0 370 880 B1**

# (12) FASCICULE DE BREVET EUROPEEN

(45) Date de publication du fascicule du brevet : **11.11.92 Bulletin 92/46**

(51) Int. Cl.$^5$ : **C07C 217/84,** A61K 7/13

(21) Numéro de dépôt : **89403182.2**

(22) Date de dépôt : **20.11.89**

(54) **Nouvelles métaphénylènediamines trialcoxy-substituées, leur procédé de préparation, et leur utilisation en tant que coupleurs pour la teinture d'oxydation des fibres kératiniques et en particulier des cheveux humains.**

(30) Priorité : **22.11.88 LU 87396**

(43) Date de publication de la demande : **30.05.90 Bulletin 90/22**

(45) Mention de la délivrance du brevet : **11.11.92 Bulletin 92/46**

(84) Etats contractants désignés : **AT BE CH DE ES FR GB GR IT LI NL SE**

(56) Documents cités : **FR-A- 2 542 193**

(73) Titulaire : **L'OREAL**
**14, Rue Royale**
**F-75008 Paris (FR)**

(72) Inventeur : **Junino, Alex**
**16, rue Docteur Bergonié**
**F-93180 Livry-Gargan (FR)**
Inventeur : **Vandenbossche, Jean-Jacques**
**6, rue Léon Richet**
**F-93600 Aulnay-sous-Bois (FR)**
Inventeur : **Borowiak, Hervé**
**84, 8ème avenue**
**F-93290 Tremblay-les-Gonesse (FR)**
Inventeur : **Lang, Gérard**
**44, avenue Lacour**
**F-95210 Saint-Gratien (FR)**

(74) Mandataire : **Casalonga, Axel et al**
**BUREAU D.A. CASALONGA - JOSSE**
**Morassistrasse 8**
**W-8000 München 5 (DE)**

**EP 0 370 880 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouvelles métaphénylènediamines, leur procédé de préparation, les compositions tinctoriales pour fibres kératiniques et en particulier pour cheveux humains contenant ces métaphénylènediamines jouant le rôle de coupleurs, associés à des précurseurs de colorants d'oxydation, ainsi qu'un procédé de teinture utilisant lesdites compositions.

On sait qu'il est courant d'utiliser pour la teinture des fibres kératiniques telles que les cheveux humains ou les fourrures, des compositions tinctoriales contenant des précurseurs de colorants d'oxydation et en particulier des paraphénylène diamines, des ortho- ou para-aminophénols que l'on désigne généralement sous le terme de bases d'oxydation.

On sait également que pour faire varier les nuances obtenues avec ces bases d'oxydation, on utilise des modificateurs de coloration ou coupleurs, et en particulier des métaphénylènediamines, des méta-aminophénols et des métadiphénols.

Dans les milieux alcalins oxydants utilisés habituellement en teinture d'oxydation, les paraphénylènediamines et para-aminophénols donnent naissance en présence de coupleurs tels que les métaphénylènediamines à des indamines ou à des indoanilines colorées.

Les indamines formées à partir de métaphénylènediamines et de para-phénylènediamines en milieu alcalin oxydant, et plus particulièrement en présence d'eau oxygénée, confèrent aux fibres kératiniques des colorations bleues très puissantes. Les indoanilines formées à partir de métaphénylènediamines et de para-aminophénols en milieu alcalin oxydant confèrent aux fibres kératiniques des colorations rouges plus ou moins pourpres. Selon les bases d'oxydation auxquelles elles sont associées, les métaphénylènediamines peuvent donc donner des colorations rouges ou bleues qui sont deux couleurs fondamentales en teinture capillaire, indispensables pour obtenir non seulement des noirs et des gris mais aussi des châtains cuivrés ou cendrés. On voit ainsi le rôle extrêmement important joué par les métaphénylènediamines en teinture capillaire d'oxydation.

Il est important par ailleurs que les bases d'oxydation et les coupleurs, qui sont utilisés dans les compositions de teinture par oxydation, confèrent aux cheveux des colorations stables à la lumière, au lavage, aux intempéries et à la transpiration. Il est souhaitable que ces colorations soient peu sélectives ou non sélectives, c'est-à-dire que les couleurs obtenues sur cheveux naturels et sensibilisés par une permanente ou une décoloration soient sensiblement identiques. Il est nécessaire également que ces composés bénéficient d'une bonne innocuité.

On connaît déjà de nombreux coupleurs du type des métaphénylènediamines substituées sur le noyau aromatique. Cependant, un grand nombre d'entre eux ne répondent pas aux exigences souhaitées.

La demanderesse vient de découvrir de nouvelles métaphénylènediamines qui allient une très bonne innocuité avec les qualités tinctoriales d'un bon coupleur et qui peuvent donc être utilisées avantageusement comme coupleurs en association avec des précurseurs de colorants d'oxydation, notamment de type para, dans des compositions de teinture d'oxydation pour fibres kératiniques.

On entend par "précurseurs de colorant d'oxydation du type para" des composés benzéniques ou hétérocycliques substitués par deux groupes amino ou par un groupe amino et un groupe hydroxyle en position para l'un par rapport à l'autre.

Associées à la plupart des paraphénylènediamines en milieu alcalin oxydant, les métaphénylènediamines selon l'invention confèrent aux cheveux des colorations bleues puissantes plus ou moins riches en vert ou en pourpre.

Lorsqu'elles sont associées aux para-aminophénols en milieu alcalin oxydant, les métaphénylènediamines selon l'invention confèrent aux cheveux des colorations rouges de bonne stabilité.

La présente invention a donc pour objet les métaphénylènediamines répondant à la formule (I) ci-dessous ou leurs sels d'addition avec un acide :

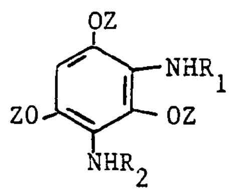 (I)

formule dans laquelle :

$R_1$ et $R_2$ représentent indépendamment l'un de l'autre un atome d'hydrogène, un radical alkyle ayant 1 à 4 atomes de carbone ou un radical mono- ou polyhydroxyalkyle ayant 2 ou 3 atomes de carbone;

Z représente un radical alkyle ayant de 1 à 4 atomes de carbone, sous réserve que lorsque $R_1$ et $R_2$ désignent simultanément un atome d'hydrogène, Z ne désigne pas le radical méthyle.

La présente invention a également pour objet le procédé de préparation des composés de formule (I).

Un autre objet de la présente invention est une composition de teinture capillaire d'oxydation comprenant, un ou plusieurs coupleurs choisis parmi les composés de formule (I) et leurs sels d'addition avec les acides, en association avec au moins un précurseur de colorant d'oxydation de type para, dans un support aqueux cosmétiquement acceptable.

La présente invention vise aussi le procédé de teinture des cheveux utilisant la composition tinctoriale ci-dessus.

Les composés de formule (I) sont préparés à partir des 2,4-dinitro-1,3,5-trialcoxybenzènes de formule (II) ci-après :

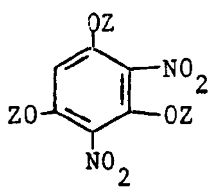

(II)

dans laquelle Z représente un radical alkyle en $C_1$-$C_4$.

Selon que les radicaux $R_1$ et $R_2$ sont identiques ou différents, les composés (I) sont préparés par l'un des schémas réactionnels suivants :

<u>1er cas $R_1$= $R_2$</u>

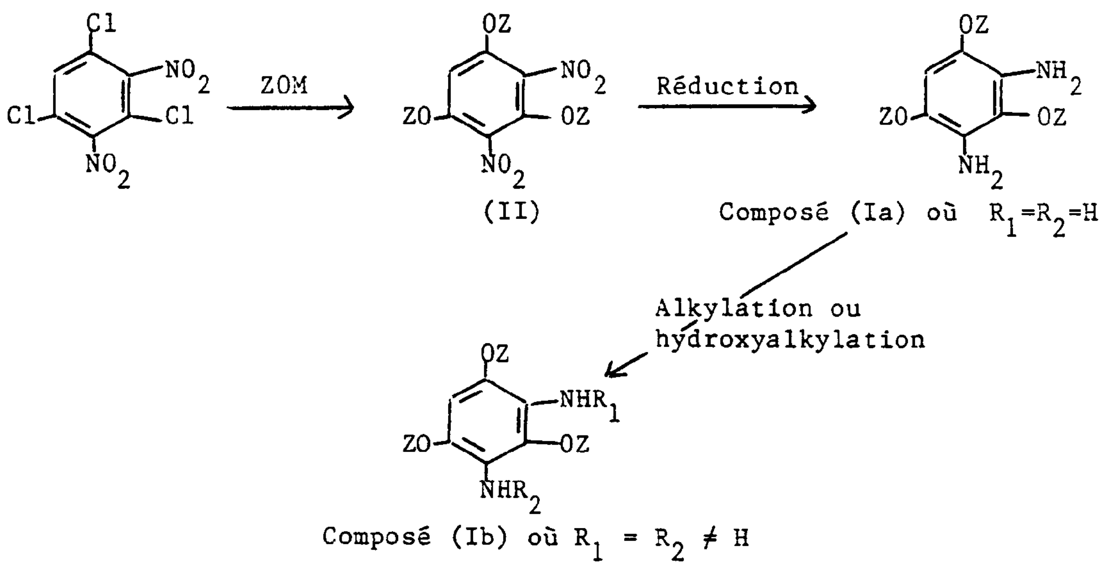


(II)

Composé (Ia) où $R_1$=$R_2$=H

Composé (Ib) où $R_1$ = $R_2$ ≠ H

Dans les formules ci-dessus $R_1$, $R_2$ et Z ont les significations ci-dessus définies.

Le 2,4-dinitro-1,3,5-trialcoxybenzène (II) est préparé à partir du 2,4-dinitro-1,3,5-trichlorobenzène par action de l'alcoolate ZOM où M représente un métal alcalin ou alcalino-terreux, en présence de l'alcool ZOH et d'un solvant tel que le toluène ou la N-méthylpyrrolidone.

Par réduction soit par le fer en présence d'acide acétique, soit par l'hydrogène en présence d'un catalyseur tel que le palladium sur charbon, on obtient le 2,4-diamino-1,3,5-trialcoxybenzène de formule (Ia) où $R_1$ et $R_2$ désignent tous deux hydrogène ($R_1$ = $R_2$ = H).

Par alkylation ou hydroxyalkylation du composé de formule (Ia) on obtient le composé de formule (Ib) où $R_1$ et $R_2$ désignent tous deux le même substituant différent de l'hydrogène ($R_1$=$R_2$≠H)

2ème cas $R_1 \neq R_2$

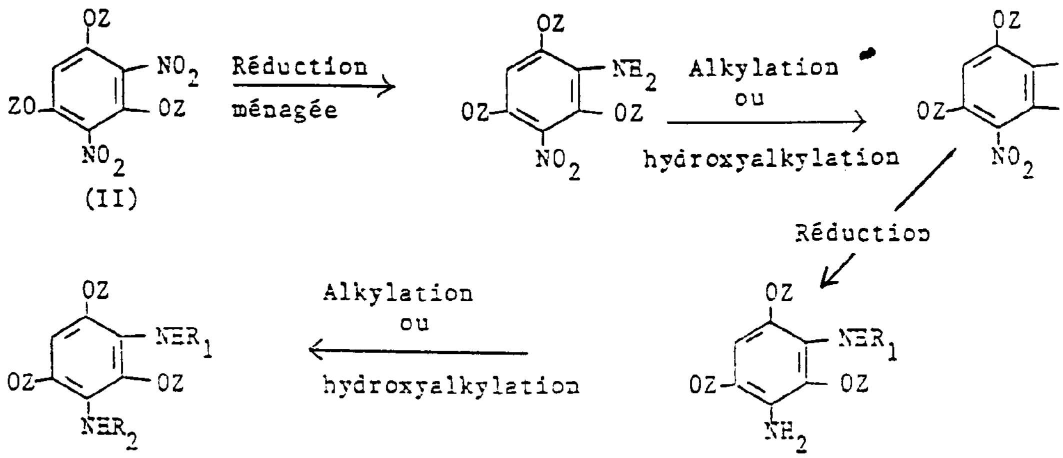


(II)

Composé (Id) $R_1 \neq R_2 \neq H$ Composé (Ic) $R_1 \neq H$ $R_2 = H$

Le 2,4-dinitro-1,3,5-trialcoxybenzène (II) est soumis dans une première étape à une réduction ménagée qui s'effectue par transfert d'hydrogène en présence d'un catalyseur tel que le palladium sur charbon et d'hypophosphite de sodium comme donneur d'hydrogène, dans le tétrahydrofurane en présence d'eau. Dans une seconde étape, le 2-amino-4-nitro-1,3,5-trialcoxybenzène est soumis à une alkylation ou hydroxyalkylation du groupement amino. Le composé ainsi obtenu est réduit par le fer en présence d'acide acétique. On obtient ainsi le composé de formule (Ic) où $R_2$ est hydrogène et $R_1$ est différent de l'hydrogène ($R_1 \neq H$ $R_2 = H$). Par alkylation ou hydroxyalkylation de ce composé de formule (Ic) on obtient le composé de formule (Id) où $R_1$ et $R_2$ sont différents l'un de l'autre et différents de l'hydrogène ($R_1 \neq R_2 \neq H$)

Les composé préférés de formule (I) sont les suivants :

- 2-(β-hydroxyéthyl)amino-4-amino-1,3,5-triméthoxybenzène,
- 2,4-diamino-1,3,5-triéthoxybenzène,
- 2-méthylamino-4-amino-1,3,5-triméthoxybenzène,
- 2-méthylamino-4-(β-hydroxyéthyl)amino-1,3,5-triméthoxybenzène,

ainsi que leurs sels d'addition avec un acide et en particulier un acide minéral tel que les acides chlorhydrique, bromhydrique ou sulfurique.

Les compositions de teinture pour cheveux conformes à l'invention comprennent, au moins un composé de formule (I) ou l'un de ses sels d'acide, en association avec au moins un précurseur de colorant d'oxydation de type para, dans un support aqueux cosmétiquement acceptable. Dans cette composition le composé de formule (I) ou son sel joue le rôle de coupleur.

Le précurseur de colorant d'oxydation de type para est choisi parmi les dérivés benzéniques ou hétérocycliques comme par exemple la pyridine sur lesquels sont fixés en position para deux groupements amino ou un groupement amino et un groupement hydroxy. Ces précurseurs de colorants d'oxydation peuvent être présents dans les compositions tinctoriales sous forme de bases libres ou sous forme de sels d'addition d'acides.

Les précurseurs de colorants d'oxydation particulièrement préférés utilisables conformément à l'invention, sont choisis parmi les paraphénylènediamines répondant à la formule générale (III) suivante:

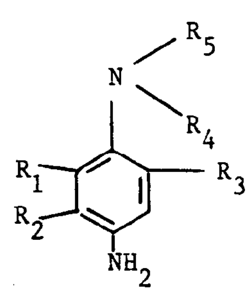

(III)

et les sels correspondants, formule dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ peuvent former conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène.

Parmi les composés de formule (III), on peut citer la p-phénylènediamine, la p-toluylènediamine, la méthoxyparaphénylènediamine, la chloroparaphénylènediamine, la 2,6-diméthyl-p-phénylènediamine, la 2,5-diméthyl-paraphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di-(β-hydroxyéthyl)paraphénylènediamine, la 3-méthyl-4-amino-N,N-di-(β-hydroxyéthyl)aniline, la 3-chloro-4-amino-N,N-di--(β-hydroxyéthyl)aniline, la 4-amino-N,N-(éthyl, carbamylméthyl) aniline, la 3-méthyl-4-amino-N,N-(éthyl, carbamylméthyl) aniline, la 4-amino-N,N-(éthyl, β-pipéridinoéthyl)aniline, la 3-méthyl-4-amino-N,N-(éthyl, β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)aniline, la 4-amino-N-β-méthoxyéthylaniline, la 3-méthyl-4-amino-N,N-(éthyl, β-acétylaminoéthyl)aniline, la 4-amino-N,N-(éthyl, β-mésylaminoéthyl)aniline, la 3-méthyl-4-amino-N,N-(éthyl, β-mésylaminoéthyl)aniline, la 4-amino-N,N-(éthyl, β-sulfoéthyl)aniline, la 3-méthyl-4-amino-N,N-(éthyl, β-sulfoéthyl)aniline, la N-[(4'-amino)phényl]morpholine, la N-[(4'-amino)phényl] pipéridine, la 2,3-diméthyl-p-phénylènediamine, l'isopropyl-p-phénylènediamine. Ces précurseurs de colorants par oxydation de type para peuvent être introduits dans la composition tinctoriale sous forme de base libre ou sous forme de sels, tels que sous forme de chlorhydrate, de bromhydrate ou de sulfate.

Le composé (I) ou ses sels peut également être utilisé avec des p-aminophénols pour donner des nuances particulièrement stables à la lumière, aux intempéries et au lavage après développement en présence d'oxydant. Parmi les para-aminophénols, on peut citer le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

Le composé (I) et ses sels peuvent également être utilisé avec des précurseurs de colorants d'oxydation para hétérocycliques parmi lesquels on peut citer la 2,5-diaminopyridine, la 2-hydroxy-5-aminopyridine, la tétra-aminopyrimidine.

Les compositions tinctoriales selon l'invention peuvent également contenir des précurseurs de colorants d'oxydation de type ortho tels que les orthoaminophénols et les orthophénylènediamines. On peut citer par exemple, le 1-amino-2-hydroxybenzène, le 6-méthyl-1-hydroxy 2-aminobenzène, le 4-méthyl-1-amino-2-hydroxybenzène.

Les compositions tinctoriales conformes à l'invention contenant le composé (I) ou ses sels peuvent contenir éventuellement d'autres coupleurs connus en eux-mêmes tels que les métadiphénols, les métaaminophénols, les métaphénylènediamines autres que celles de formule (I), les métaacylaminophénols, les métauréidophénols, les métacarbalcoxyaminophénols, l'α-naphtol, les coupleurs possédant un groupe méthylène actif tels que les composés β-cétoniques et les pyrazolones.

On peut citer en particulier, à titre d'exemple, le 2,4-dihydroxyphénoxyéthanol, le 2,4-dihydroxyanisole, le métaaminophénol, la résorcine, le monométhyléther de résorcine, le 2-méthyl-5-aminophénol, le 2-méthyl-5-N-(β-hydroxyéthyl) aminophénol, le 2-méthyl-5-N-(β-mésylamino-éthyl)aminophénol, le 2,6-diméthyl-3-aminophénol, la 6-hydroxybenzomorpholine, le 2,4-diaminophénoxyéthanol, la 6-aminobenzomorpholine, le 2-[N-(β-hydroxyéthyl)amino]-4-aminophénoxyéthanol, le 2-amino-4-N-(β-hydroxyéthyl)aminoanisole, le (2,4-diamino)phényl-β,γ-dihydroxypropyléther, la 2,4-diaminophénoxyéthylamine, le 3,4-méthylènedioxyphénol, la 3,4-méthylènedioxyaniline et leurs sels.

On peut rajouter à ces compositions, comme cela est bien connu en vue de nuancer ou enrichir en reflets les colorations apportées par les précurseurs de colorants d'oxydation, des colorants directs tels que des colorants azoïques, anthraquinoniques ou les dérivés nitrés de la série benzénique.

L'ensemble des composés para et des coupleurs utilisés dans les compositions tinctoriales conformes à l'invention représente de préférence de 0,1 à 7% en poids du poids total de ladite composition. La concentration en composé (I) peut varier entre 0,05 et 3,5% du poids total de la composition.

Le support aqueux cosmétiquement acceptable a un pH qui peut varier entre 8 et 11, et il est de préférence compris entre 9 et 11.

Il est ajusté à la valeur désirée à l'aide d'un agent alcalinisant tels que l'ammoniaque, les carbonates alcalins, les alcanolamines comme la mono-, la di- ou la triéthanolamine.

Les compositions tinctoriales conformes à l'invention contiennent également dans leur forme de réalisation préférée des agents tensio-actifs anioniques, cationiques, non-ioniques, amphotères ou leurs mélanges. Parmi ces agents tensio-actifs, on peut citer plus particulièrement les alkylbenzènesulfonates, les alkylnaphtalènesulfonates, les sulfates, les éther-sulfates et sulfonates d'alcools gras, les sels d'ammonium quaternaire tels que le bromure de triméthylcétylammonium, le bromure de cétylpyridinium, les éthanolamides d'acides gras éventuellement oxyéthylénés, les acides, les alcools et les amines polyoxyéthylénés, les alcools polyglycérolés, les alkylphénols polyoxyéthylénés ou polyglycérolés ainsi que les alkylsulfates polyoxyéthylénés. Les agents tensio-actifs sont présents dans les compositions conformes à l'invention dans des proportions comprises entre 0,5 et 40% en poids, et de préférence entre 4 et 30% en poids par rapport au poids total de la composition.

Ces compositions peuvent également contenir des solvants organiques pour solubiliser les composés qui ne seraient pas suffisamment solubles dans l'eau. Parmi ces solvants, on peut mentionner à titre d'exemple, les alcanols inférieurs en $C_1$-$C_4$ tels que l'éthanol et l'isopropanol, les alcools aromatiques comme l'alcool benzylique et l'alcool phényl-éthylique; le glycérol; les glycols ou éthers de glycol comme le 2-butoxyéthanol, l'éthylèneglycol, le propylèneglycol, le monoéthyléther- et le monométhyléther du diéthylèneglycol, ainsi que les produits analogues et leurs mélanges. Les solvants sont présents de préférence dans une proportion comprise entre 1 et 40% en poids, et en particulier entre 5 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose tels que la méthylcellulose, l'hydroxyéthylcellulose, l'hydroxypropylcellulose, l'hydroxyméthylcellulose, la carboxyméthylcellulose, les polymères d'acide acrylique, la gomme de Xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids par rapport au poids total de la composition.

Les compositions peuvent contenir des agents anti-oxydants choisis en particulier parmi le sulfite de sodium, l'acide thioglycolique, le bisulfite de sodium, l'acide ascorbique et l'hydroquinone. Ces agents anti-oxydants sont présents dans la composition dans des proportions comprises entre 0,05 et 1,5% en poids par rapport au poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses telles que sous forme de liquides, de crèmes, de gels ou sous toute autre forme appropriée pour réaliser une teinture de fibres kératiniques et notamment des cheveux humains. Elles peuvent également être conditionnées en flacons aérosols en présence d'un agent propulseur.

Les compositions tinctoriales conformes à l'invention, contenant un précurseur de colorant par oxydation de type para et le composé (I) ou l'un de ses sels, sont utilisées dans un procédé de teinture capillaire par oxydation.

Conformément à ce procédé, on mélange au moment de l'emploi la composition tinctoriale décrite ci-dessus avec une solution oxydante en une quantité suffisante, puis on applique le mélange obtenu sur les cheveux.

La solution oxydante contient des agents d'oxydation tels que l'eau oxygénée, le peroxyde d'urée ou les persels tels que le persulfate d'ammonium. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Un autre procédé de mise en oeuvre du composé (I) conforme à l'invention consiste à teindre les cheveux suivant un procédé en plusieurs temps, selon lequel, dans un premier temps, on applique le précurseur de colorant d'oxydation para au moyen d'une composition sus-définie et dans un deuxième temps, on applique le composé (I). L'agent oxydant est présent dans la composition appliquée dans le deuxième temps ou bien est appliqué sur les cheveux eux-mêmes dans un troisième temps, chacune des compositions appliquées en premier et en deuxième temps et, s'il y a lieu l'agent oxydant appliqué dans un troisième temps, sont laissés en contact des cheveux pendant 10 à 40 minutes, après quoi on rince les cheveux, on les lave au shampooing, on les rince à nouveau et on les sèche.

Les exemples ci-après servent à mieux illustrer l'invention mais ne sont en aucun cas limitatifs de sa portée.

EXEMPLE DE PREPARATION N° 1

Préparation du dichlorhydrate de 2-(β-hydroxyéthyl)amino-4-amino-1,3,5-triméthoxybenzène

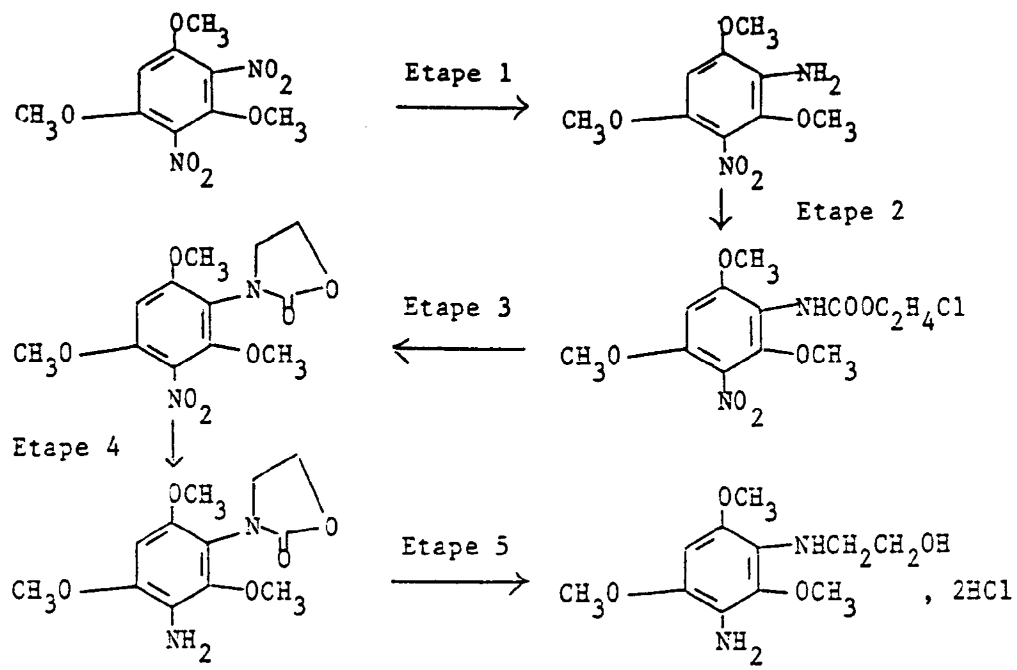


Etape 1

Préparation du 2-amino-4-nitro-1,3,5-triméthoxybenzène

On chauffe au reflux une suspension de 26 g de palladium sur charbon (10%) dans 1,3 l de tétrahydrofurane et 26 ml d'eau contenant 1 mole (258 g) de 2,4-dinitro-1,3,5-triméthoxybenzène. On ajoute à cette suspension une solution de 247 g d'hypophosphite de sodium dans 260 ml d'eau en 3 heures. On maintient le reflux 30 minutes supplémentaires. Après refroidissement, le catalyseur est éliminé par filtration, et la phase aqueuse par décantation. La phase organique est concentrée sous vide puis versée sur de la glace. Le produit attendu précipite. Il fond à 111°C.

L'analyse du produit obtenu recristallisé de l'éthanol à 96° donne les résultats suivants :

| | Calculé pour $C_9H_{12}N_2O_5$ | Trouvé |
|---|---|---|
| C% | 47,37 | 47,31 |
| H% | 5,26 | 5,23 |
| N% | 12,28 | 12,36 |
| O% | 35,09 | 34,89 |

Etape 2

Préparation du 2-(β-chloréthoxycarbonyl)amino-4-nitro-1,3,5-triméthoxybenzène

On dissout 0,26 mole (60 g) de 2-amino-4-nitro-1,3,5-triméthoxybenzène dans 270 ml de dioxane. On ajoute 26,3 g de carbonate de calcium, puis on élève la température aux environs de 90°C. On introduit alors sous agitation 0,34 mole (48,6 g) de chloroformiate de - chloréthyle. L'addition terminée, on maintient l'agitation 30 minutes supplémentaires à 90°C. Le milieu réactionnel est dilué par 600 ml d'eau glacée puis acidifié par ajout de 25 ml d'acide chlorhydrique concentré. Le précipité obtenu est essoré, lavé à l'eau. Après séchage, il fond à 133°C.

L'analyse du produit obtenu, recristallisé de l'isopropanol, donne les résultats suivants :

| | Calculé pour $C_{12}H_{15}N_2O_7Cl$ | Trouvé |
|---|---|---|
| C% | 43,05 | 42,90 |
| H% | 4,48 | 4,32 |
| N% | 8,37 | 8,51 |
| O% | 33,48 | 33,21 |
| Cl% | 10,61 | 10,53 |

Etape 3

Préparation de la N-[(3′-nitro-2′,4′,6′-triméthoxy)phényl] oxazolidine-1,3 one-2

On chauffe à 65°C 0,23mole (80 g) de carbamate de β-chloréthyle préparé à l'étape précédente dans 240 ml de méthanol. On ajoute 0,25 mole de méthylate de sodium en solution à 30% dans le méthanol. Le chauffage est maintenu 15 minutes supplémentaires après la fin de l'addition. Le mélange réactionnel est dilué par 250 ml d'eau glacée. Le produit attendu précipite. Après réempâtage dans 60 ml d'éthanol, puis séchage, il fond à 178°C.

L'analyse du produit obtenu, recristallisé de l'éthanol, donne les résultats suivants :

| | Calculé pour $C_{12}H_{14}N_2O_7$ | Trouvé |
|---|---|---|
| C% | 48,32 | 48,37 |
| H% | 4,70 | 4,91 |
| N% | 9,39 | 9,48 |
| O% | 37,58 | 37,50 |

Etape 4

Préparation de la N-[(3′-amino-2′,4′,6′triméthoxy)phényl] oxazolidine-1,3 one-2

A 360 ml d'eau additionnés de 6 ml d'acide acétique et préalablement chauffés au bain-marie bouillant, on ajoute 120 g de fer en poudre réduit à l'hydrogène et peu à peu, sous agitation, 0,2 mole (60 g) de l'oxazolidine-1,2 one-3 préparée à l'étape précédente. L'addition terminée, on maintient le chauffage pendant 30 minutes supplémentaires. Après refroidissement, le milieu réactionnel est centrifugé. Les boues ferriques contenant le produit attendu sont lavées à l'eau. Le produit attendu est extrait des boues ferriques par l'acétone. Après recristallisation de l'isopropanol, il fond à 162°C.

L'analyse élémentaire du produit obtenu donne les résultats suivants :

| | Calculé pour $C_{12}H_{16}N_2O_5$ | Trouvé |
|---|---|---|
| C% | 53,73 | 53,64 |
| H% | 5,97 | 6,01 |
| N% | 10,45 | 10,68 |
| O% | 29,85 | 30,07 |

Etape 5

Préparation du dichlorhydrate de 2-(β-hydroxyéthyl)amino-4-amino-1,3,5-triméthoxybenzène

On chauffe au reflux 0,1 mole (28 g) de l'oxazolidine-1,3 one-2 préparée à l'étape précédente dans 100

ml de soude 10N additionnés de 30 ml d'eau et 70 ml d'éthanol. Le milieu réactionnel est refroidi après 7 heures 30 minutes de chauffage. L'alcool est éliminé par évaporation. Le produit attendu est extrait des eaux résiduelles à l'acétate d'éthyle. Par ajout d'une solution d'éthanol chlorhydrique, le chlorhydrate précipite du milieu réactionnel.

L'analyse du produit obtenu donne les résultats suivants :

| | Calculé pour $C_{11}H_{20}N_2O_4Cl_2$ | Trouvé |
|---|---|---|
| C% | 41,91 | 41,83 |
| H% | 6,35 | 6,36 |
| N% | 8,89 | 9,03 |
| O% | 20,32 | 20,06 |
| Cl% | 22,52 | 22,37 |

EXEMPLE DE PREPARATION N° 2

Préparation du 2,4-diamino 1,3,5-triéthoxybenzène

Etape 1

Préparation du 2,4-dinitro-1,3,5-triéthoxybenzène

On porte à 70°C le mélange constitué par 0,18 mole (50 g) de 2,4-dinitro-1,3,5-trichlorobenzène et 250 ml de dioxanne. On introduit alors sous agitation 0,64 mole (219,9 g) d'éthylate de sodium en solution à 20% dans l'éthanol. L'addition terminée, on maintient l'agitation deux heures supplémentaires à 70°C. Du milieu réactionnel refroidi et dilué à l'eau glacée, on isole par filtration le produit attendu qui a cristallisé. Après séchage, il est recristallisé de l'éthanol. Il fond alors à 105°C.

L'analyse du produit obtenu donne les résultats suivants :

| | Calculé pour $C_{12}H_{16}N_2O_7$ | Trouvé |
|---|---|---|
| C% | 48,00 | 48,02 |
| H% | 5,33 | 5,41 |
| N% | 9,33 | 9,41 |
| O% | 37,33 | 37,13 |

Etape 2

Préparation du dichlorohydrate de 2,4-diamino-1,3,5-triméthoxybenzène

Dans un autoclave, on chauffe à 80°C pendant 1 heure en présence de 4 g de palladium à 10% sur charbon et sous pression d'hydrogène 1960 kPa (20 kg/cm$^3$) le mélange constitué par 0,1 mole (31,3 g) de 2,4-dinitro-1,3,5-triméthoxybenzène, 120 ml de diglyme et 6 ml d'eau.

Le catalyseur est éliminé par filtration à chaud du milieu réactionnel.

Le produit attendu précipite du filtrat après addition de 50 ml d'acide chlorhydrique concentré. Le produit ainsi préparé est purifié, après lavage et séchage à chaud sous vide d'air, par dissolution dans 50 ml d'eau bouillante, filtration de la solution aqueuse chaude, et précipitation par ajout de 10 ml d'acide chlorhydrique concentré.

L'analyse du produit obtenu donne les résultats suivants :

| | Calculé pour $C_{12}H_{22}N_2O_3Cl_2$ | Trouvé |
|---|---|---|
| C% | 46,01 | 46,08 |
| H% | 7,03 | 7,07 |
| N% | 8,94 | 8,78 |
| O% | 15,33 | 15,33 |
| Cl% | 22,68 | 22,80 |

EXEMPLE DE PREPARATION N° 3

Préparation du 2-méthylamino 4-amino 1,3,5-triméthoxybenzène

Etape 1

Préparation du 2-formylamino-4-nitro-1,3,5-triméthoxybenzène

On chauffe au reflux le mélange constitué par 0,11 mole (26,6 g) de 2-amino-4-nitro-1,3,5-triméthoxybenzène et de 55 ml d'acide formique, pendant trois heures. Le milieu réactionnel est refroidi et dilué à l'eau glacée. Le produit attendu précipite, il est isolé par filtration. Après séchage, il est recristallisé de l'alcool éthylique. Il fond à 98°C.

L'analyse du produit obtenu donne les résultats suivants :

| | Calculé pour $C_{10}H_{12}N_2O_6$ | Trouvé |
|---|---|---|
| C% | 46,88 | 47,01 |
| H% | 4,72 | 4,74 |
| N% | 10,93 | 10,89 |
| O% | 37,47 | |

Etape 2

Préparation du 2-[(N-formyl-N-méthyl)]amino)-4-nitro-1,3,5-triméthoxybenzène

On dissout 0,058 mole (15 g) de 2-formylamino-4-nitro-1,3,5-triméthoxybenzène dans 75 ml de diméthylformamide à température ambiante. On ajoute à ce mélange 0,063 mole (11,84 g) de méthylate de sodium en solution à 30% dans le méthanol. On ajoute goutte à goutte, à une température comprise entre 20°C et 45°C 0,063 mol (7,94 g) de diméthylsulfate. L'addition terminée, on maintient l'agitation 15 minutes supplémentaires à 45°C. Le mélange est dilué par 600 ml d'eau glacée. Le produit est extrait à l'acétate d'éthyle. Après décantation de la phase aqueuse la phase organique est séchée sur sulfate de sodium et concentrée sous vide. Après réempâtage dans l'éther isopropylique et séchage, le produit attendu fond à 110°C.

L'analyse du produit obtenu donne les résultats suivants :

| | Calculé pour $C_{11}H_{14}N_2O_6$ | Trouvé |
|---|---|---|
| C% | 48,89 | 48,69 |
| H% | 5,22 | 5,22 |
| N% | 10,37 | 10,34 |
| O% | 35,52 | |

Etape 3

Préparation du dichlorhydrate de 2-méthylamine-4-amino-1,3,5-triméthoxybenzène

On porte à reflux le suspension constituée de 8 g de zinc en poudre, de 0,2 g de chlorure d'ammonium, de 15 ml d'éthanol et de 5 ml d'eau. On introduit peu à peu 7,4 $10^{-3}$moles (2 g) de 2-[(N-formyl-N-méthyl) amino)]-4-nitro-1,3,5-triméthoxybenzène. L'addition terminée, on maintient le chauffage pendant cinq minutes. Les sels de zinc sont éliminés du milieu réactionnel par filtration. Le filtrat est dilué par 50 ml d'acide chlorhydrique concentré et chauffé au bain marie bouillant. Le mélange réactionnel est évaporé sous vide, le résidu est redissout et on isole le produit attendu.

L'analyse du produit obtenu donne les résultats suivants :

| | Calculé pour $C_9H_{18}N_2O_3Cl_2$ | Trouvé |
|---|---|---|
| C% | 39,57 | |
| H% | 6,64 | |
| N% | 10,26 | |
| O% | 17,57 | |
| Cl% | 25,96 | |

## Exemple de teinture 1

On prépare le mélange tinctorial suivant :

| | | |
|---|---|---|
| - p-phénylènediamine | | 0,27 g |
| - dichlorhydrate de 2-(β-hydroxyéthyl)amino-4 amino-1,3,5-triméthoxy benzène | | 0,78 g |
| - Alcool oléique polyglycérolé à 2 moles de glycérol | | 4,5 g |
| - Alcool oléique polyglycérolé à 4 moles de glycérol | | 4,5 g |
| - "ETHOMEEN O 12" - Société ARMOUR HESS CHEMICAL LTD. (oléylamine oxyéthylénée à 12 moles d'O.E.) | | 4,5 g |
| - "COMPERLAN KD" - Société HENKEL (diéthanolamide de coprah) | | 9 g |
| - Propylène glycol | | 4 g |
| - 2-butoxyéthanol | | 8 g |
| - Ethanol à 96° | | 6 g |
| - "MASQUOL DTPA" - Société PROTEX (sel pentasodique de l'acide diéthylène triamine pentacétique) | | 2 g |
| - Hydroquinone | | 0,15 g |
| - Solution de bisulfite de sodium à 35° Bé | | 1,3 g |
| - Ammoniaque à 22°Bé | | 10 g |
| - Eau | qsp | 100 g |
| - pH : 10,2 | | |

Au moment de l'emploi, on ajoute 100 g d'eau oxygénée à 20 volumes. Le mélange, appliqué 20 minutes à 35°C sur cheveux naturellement blancs à 90%, leur confère après shampooing et rinçage, une coloration gris myrtille.

Exemple de teinture 2

On prépare le mélange tinctorial suivant :

| | | |
|---|---|---|
| - p-aminophénol | | 0,545 g |
| - Dichlorhydrate de 2-(β-hydroxyéthyl)amino-4-amino-1,3,5-triméthoxybenzène | | 1,57 g |
| - Alcool oléique polyglycérolé à 2 moles de glycérol | | 4,5 g |
| - Alcool oléique polyglycérolé à 4 moles de glycérol | | 4,5 g |
| - "ETHOMEEN O 12" - Société ARMOUR HESS CHEMICAL LTD. (oléylamine oxyéthylénée à 12 moles d'O.E.) | | 4,5 g |
| - "COMPERLAN KD" - Société HENKEL (diéthanolamide de coprah) | | 9 g |
| - Propylène glycol | | 4 g |
| - 2-butoxyéthanol | | 8 g |
| - Ethanol à 96° | | 6 g |
| - "MASQUOL DTPA" - Société PROTEX (sel pentasodique de l'acide diéthylène triamine pentacétique) | | 2 g |
| - Hydroquinone | | 0,15 g |
| - Solution de bisulfite de sodium à 35° Bé | | 1,3 g |
| - Ammoniaque à 22°Bé | | 10 g |
| - Eau | qsp | 100 g |
| - pH : 10,2 | | |

Au moment de l'emploi, on ajoute 100 g d'eau oxygénée à 20 volumes. Le mélange, appliqué 20 minutes à 35°C sur cheveux décolorés, leur confère, après shampooing et rinçage, une coloration framboise.

Exemple de teinture 3

On prépare le mélange tinctorial suivant :

| | | |
|---|---|---|
| - Dichlorhydrate de 2,4-diamino-1,3,5-triéthoxybenzène | | 0,78 g |
| - p-phénylènediamine | | 0,27 g |
| - Alcool oléique polyglycérolé à 2 moles de glycérol | | 4,5 g |
| - Alcool oléique polyglycérolé à 4 moles de glycérol | | 4,5 g |
| - "ETHOMEEN O 12" - Société ARMOUR HESS CHEMICAL LTD. (oléylamine oxyéthylénée à 12 moles d'oxyde d'éthylène) | | 4,5 g |
| - "COMPERLAN KD" - Société HENKEL (diéthanolamide de coprah) | | 9 g |
| - Propylène glycol | | 4 g |
| - 2-butoxyéthanol | | 8 g |
| - Ethanol à 96° | | 6 g |
| - "MASQUOL DTPA" - Société PROTEX (sel pentasodique de l'acide diéthylène triamine pentacétique) | | 2 g |
| - Hydroquinone | | 0,15 g |
| - Solution de bisulfite de sodium à 35° Baumé | | 1,3 g |
| - Ammoniaque à 22°Baumé | | 10 g |
| - Eau | qsp | 100 g |
| - pH : 10,2 | | |

Au moment de l'emploi, on ajoute 100 g d'eau oxygénée à 20 volumes. Le mélange, appliqué 20 minutes à 35 sur cheveux décolorés, leur confère, après shampooing et rinçage, une coloration bleu pourpre foncé.

Exemple de teinture 4

On prépare le mélange tinctorial suivant :

| | | |
|---|---|---|
| - p-phénylènediamine | | 0,27 g |
| - dichlorhydrate de 2-méthylamino-4-amino-1,3,5-triméthoxy benzène | | 0,71 g |
| - Alcool oléique polyglycérolé à 2 moles de glycérol | | 4,5 g |
| - Alcool oléique polyglycérolé à 4 moles de glycérol | | 4,5 g |
| - "ETHOMEEN O 12" - Société ARMOUR HESS CHEMICAL LTD. (oléylamine oxyéthylénée à 12 moles d'oxyde d'éthylène) | | 4,5 g |
| - "COMPERLAN KD" - Société HENKEL (diéthanolamide de coprah) | | 9 g |
| - Propylène glycol | | 4 g |
| - 2-butoxyéthanol | | 8 g |
| - Ethanol à 96° | | 6 g |
| - "MASQUOL DTPA" - Société PROTEX (sel pentasodique de l'acide diéthylène triamine pentacétique) | | 2 g |
| - Hydroquinone | | 0,15 g |
| - Solution de bisulfite de sodium à 35° Baumé | | 1,3 g |
| - Ammoniaque à 22°Baumé | | 10 g |
| - Eau | qsp | 100 g |
| - pH : 10,2 | | |

Au moment de l'emploi, on ajoute 100 g d'eau oxygénée à 20 volumes. Le mélange, appliqué 20 minutes à 35°C sur cheveux décolorés, leur confère après shampooing et rinçage, une coloration framboise.

**Revendications**

**Revendications pour les Etats contractants suivants: AT, BE, CH, DE, FR, GB, GR, IT, LI, NL, SE**

**1.** 1,3,5-trialcoxy métaphénylènediamine de formule :

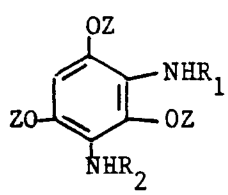

(I)

dans laquelle :

$R_1$ et $R_2$ représentent indépendamment l'un de l'autre un atome d'hydrogène, un radical alkyle ayant 1 à 4 atomes de carbone ou un radical mono- ou polyhydroxyalkyle ayant 2 ou 3 atomes de carbone;

Z représente un radical alkyle ayant 1 à 4 atomes de carbone, sous réserve que lorsque $R_1$ et $R_2$ désignent simultanément un atome d'hydrogène, Z ne désigne pas le radical méthyle, et les sels d'addition avec un acide de ce composé.

2. Composé selon la revendication 1, caractérisé par le fait qu'il est choisi parmi le 2-(β-hydroxyéthyl)amino-4-amino-1,3,5-triméthoxybenzène, le 2,4-diamino-1,3,5-triéthoxybenzène, le 2-méthylamino-4-amino-1,3, 5-triméthoxybenzène, le 2-méthylamino-4-(β-hydroxyéthyl)amino-1,3,5-triméthoxybenzène, et leurs sels d'addition avec un acide.

3. Procédé de préparation du composé de formule (I) selon la revendication 1 dans lequel $R_1$ et $R_2$ ont des significations identiques, caractérisé par le fait qu'on procède à la réduction du 2,4-dinitro-1,3,5-trialcoxybenzène de formule (II) :

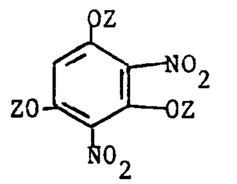

(II)

dans laquelle Z représente un radical alkyle en $C_1$-$C_4$, par le fer en présence d'acide acétique ou par l'hydrogène en présence d'un catalyseur tel que le palladium sur charbon, pour obtenir le 2,4-diamino-1,3,5-trialcoxybenzène de formule (I) suivante :

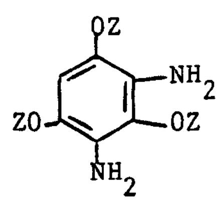

dans laquelle Z représente un radical alkyle en $C_1$-$C_4$ et si l'on désire on soumet ce composé à une alkylation ou une hydroxyalkylation pour obtenir le composé de formule suivante :

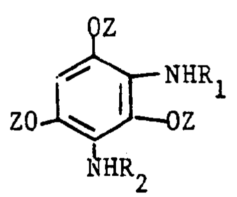

(I)

4. Procédé de préparation d'un composé de formule (I) selon la revendication 1 dans laquelle $R_1$ et $R_2$ ont des significations différentes, caractérisé par le fait que dans une première étape, on soumet le 2,4-dinitro-1,3,5-trialcoxybenzène de formule (II) :

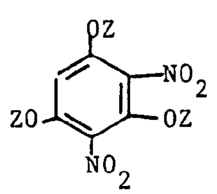

(II)

dans laquelle Z est un radical alkyle en $C_1$-$C_4$, à une réduction ménagée qui s'effectue par transfert d'hydrogène en présence d'un catalyseur tel que le palladium sur charbon et d'hypophosphite de sodium comme donneur d'hydrogène, dans le tétrahydrofurane en présence d'eau pour obtenir le composé de formule :

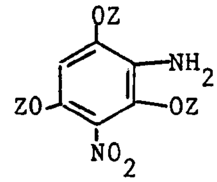

dans une seconde étape, on soumet le 2-amino-4-nitro-1,3,5-trialcoxybenzène obtenu à une alkylation ou hydroxyalkylation du groupement amino pour obtenir le composé de formule :

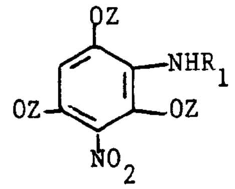

où $R_1$ est un radical alkyle ayant 1 à 4 atomes de carbone ou mono-ou polyhydroxyalkyle ayant 2 ou 3 atomes de carbone, dans une troisième étape, on réduit le composé ainsi obtenu par le fer en présence d'acide acétique pour obtenir le composé de formule :

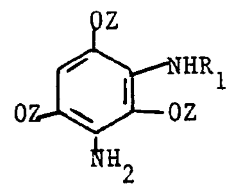

(I)

dans laquelle $R_1$ a la même signification que ci-dessus et si l'on désire on soumet ce composé à une alkylation ou une hydroxyalkylation pour obtenir le composé de formule (I ) :

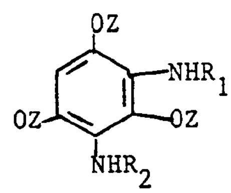

(I)

dans laquelle $R_1$ et $R_2$ sont différents entre eux et désignent un radical alkyle ayant 1 à 4 atomes de carbone ou un radical mono- ou polyhydroxyalkyle ayant 2 ou 3 atomes de carbone.

5. Composition tinctoriale pour cheveux, caractérisée par le fait qu'elle contient, dans un support aqueux cosmétiquement acceptable, un ou plusieurs composés de formule (I) selon la revendication 1 ou 2 ou leurs sels d'addition avec les acides, en association avec au moins un précurseur de colorant d'oxydation de type para.

6. Composition tinctoriale selon la revendication 5, caractérisée par le fait qu'elle contient 0,05 à 3,5% en poids du composé de formule (I) ou de l'un de ses sels d'addition avec un acide, sur la base du poids total de la composition.

7. Composition tinctoriale selon la revendication 5, caractérisée par le fait que le précurseur de colorant

d'oxydation de type para est choisi parmi les p-phénylènediamines, les p-aminophénols, les composés p-hétérocycliques et leurs mélanges.

8. Composition tinctoriale selon la revendication 7, caractérisée par le fait que les p-phénylènediamines répondent à la formule :

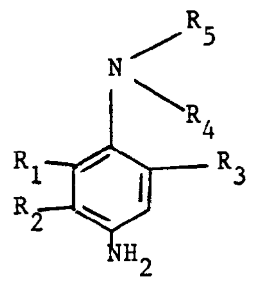

(III)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou sont les sels des composés de formule (III) ci-dessus.

9. Composition tinctoriale selon la revendication 8, caractérisée par le fait qu'elle contient au moins une paraphénylènediamine choisie parmi la p-phénylènediamine, la p-toluylènediamine, la méthoxyparaphénylènediamine, la chloroparaphénylènediamine, la 2,6-diméthyl-p-phénylènediamine, la 2,5-diméthyl-paraphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di-(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl)aniline, la 3-méthyl-4-amino-N,N-(éthyl, carbamylméthyl)aniline, la 4-amino-N,N-(éthyl, β-pipéridinoéthyl)aniline, la 3-méthyl-4-amino-N,N-(éthyl,β-pipéridinoéthyl)aniline, la 4-amino-N,N-(éthyl, β-morpholinoéthyl)aniline, la 3-méthyl-4-amino-N,N-(éthyl, -morpholinoéthyl)aniline, la 4-amino-N,N-(éthyl, β-acétylaminoéthyl)aniline, la 4-amino-N-β-méthoxyéthylaniline, la 3-méthyl-4-amino-N,N-(éthyl, β-acétylaminoéthyl)aniline, la 4-amino-N,N-(éthyl, β-mésylaminoéthyl)aniline, la 3-méthyl-4-amino-N,N-(éthyl, β-mésylaminoéthyl)aniline, la 4-amino-N,N-(éthyl, β-sulfoéthyl)aniline, la 3-méthyl-4-amino-N,N-(éthyl, β-sulfoéthyl)aniline, la N-[(4'-amino)phényl]morpholine, la N-[(4'-amino)phényl] pipéridine, la 2,3-diméthyl-p-phénylènediamine, l'isopropyl-p-phénylènediamine, sous forme de base libre ou sous forme de sel cosmétiquement acceptable.

10. Composition tinctoriale selon la revendication 7, caractérisée par le fait qu'elle contient au moins un p-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

11. Composition tinctoriale selon la revendication 7, caractérisée par le fait que le précurseur de colorant d'oxydation de type para est un composé para hétérocyclique choisi parmi la 2,5-diamino-pyridine, la 2-hydroxy-5-amino-pyridine et la tétraaminopyrimidine.

12. Composition tinctoriale selon l'une quelconque des revendications 5 à 11, caractérisée par le fait qu'elle contient également d'autres coupleurs choisis parmi les métadiphénols, les métaaminophénols, les métaphénylènediamines autres que celles de formule (I), les méta-acylamino-phénols, les métauréidophénols, les métacarbalcoxyaminophénols, l'α-naphtol, les composés β-cétoniques et les pyrazolones.

13. Composition tinctoriale selon l'une quelconque des revendications 5 à 12, caractérisée par le fait que le

poids des précurseurs de colorants d'oxydation de type para et des coupleurs est de 0,1 à 7% en poids du poids total de la composition.

**14.** Composition tinctoriale selon l'une quelconque des revendications 5 à 13, caractérisée par le fait qu'elle contient également des précurseurs de colorant de type ortho choisis parmi les orthoaminophénols et les orthophénylènediamines.

**15.** Composition tinctoriale selon l'une quelconque des revendications 5 à 14, caractérisée par le fait qu'elle contient également des colorants directs choisis parmi les colorants azoïques, anthraquinoniques et les dérivés nitrés de la série benzénique.

**16.** Composition tinctoriale selon l'une quelconque des revendications 5 à 15, caractérisée par le fait qu'elle a un pH compris entre 8 et 11, et de préférence compris entre 9 et 11.

**17.** Composition tinctoriale selon l'une quelconque des revendications 5 à 16, caractérisée par le fait qu'elle contient de 1 à 40% en poids d'un solvant organique choisi parmi les alcanols inférieurs, les alcools aromatiques, le glycérol, les glycols ou éthers de glycols, et leurs mélanges.

**18.** Composition tinctoriale selon l'une quelconque des revendications 5 à 17, caractérisée par le fait qu'elle contient également de 0,5 à 40% en poids, d'au moins un agent tensio-actif anionique, cationique, non ionique, amphotère ou leurs mélanges.

**19.** Composition tinctoriale selon l'une quelconque des revendications 5 à 18, caractérisée par le fait qu'elle contient également des adjuvants cosmétiques choisis parmi les épaississants, les agents anti-oxydants, les agents de pénétration, les agents séquestrants, les tampons, les parfums, les agents alcalinisants et les propulseurs.

**20.** Procédé de teinture capillaire par oxydation, caractérisé par le fait qu'il consiste à mélanger au moment de l'emploi la composition tinctoriale selon l'une quelconque des revendications 5 à 19 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à l'eau et à les sécher.

**21.** Procédé de teinture capillaire par oxydation, caractérisé par le fait qu'il consiste dans un premier temps à appliquer sur les cheveux une composition tinctoriale contenant au moins un précurseur de colorant d'oxydation de type para tel que défini dans l'une quelconque des revendications 7 à 11 puis, dans un deuxième temps, à appliquer une composition tinctoriale contenant au moins un composé de formule (I) ou l'un de ses sels d'addition avec un acide, l'agent oxydant étant présent dans la composition appliquée dans le deuxième temps ou bien est appliqué sur les cheveux eux-mêmes dans un troisième temps, chacune des compositions appliquées en premier et en deuxième temps et, s'il y a lieu, l'agent oxydant appliqué dans un troisième temps sont laissés en contact des cheveux pendant 10 à 40 minutes, après quoi on rince les cheveux, on les lave au shampooing, on les rince à nouveau et on les sèche.

**Revendications pour l'Etat contractant suivant : ES**

**1.** Procédé de préparation de 1,3,5-trialcoxy métaphénylènediamines de formule :

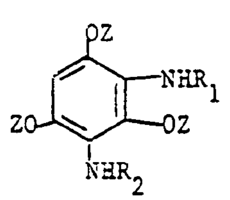

(I)

dans laquelle :

$R_1$ et $R_2$ représentent indépendamment l'un de l'autre un atome d'hydrogène, un radical alkyle ayant 1 à 4 atomes de carbone ou un radical mono- ou polyhydroxyalkyle ayant 2 ou 3 atomes de carbone;

Z représente un radical alkyle ayant 1 à 4 atomes de carbone, sous réserve que lorsque $R_1$ et $R_2$

désignent simultanément un atome d'hydrogène, Z ne désigne pas le radical méthyle, et les sels d'addition avec un acide de ce composé, caractérisé par le fait que (A) lorsque $R_1$ et $R_2$ ont des significations identiques, on procède à la réduction du 2,4-dinitro-1,3,5-trialcoxybenzène de formule (II) :

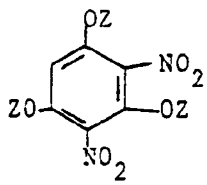

(II)

dans laquelle Z représente un radical alkyle en $C_1$-$C_4$, par le fer en présence d'acide acétique ou par l'hydrogène en présence d'un catalyseur tel que le palladium sur charbon, pour obtenir le 2,4-diamino-1,3,5-trialcoxybenzène de formule (I) suivante :

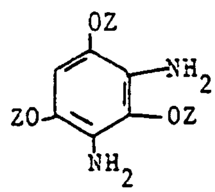

(I)

dans laquelle Z représente un radical alkyle en $C_1$-$C_4$ et si l'on désire on soumet ce composé à une alkylation ou une hydroxy alkylation pour obtenir le composé de formule :

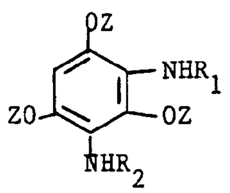

(I)

où $R_1$ et $R_2$ désignent tous deux un radical alkyle ayant de 1 à 4 atomes de carbone ou un radical mono ou polyhydroxyalkyl ayant 2 ou 3 atomes de carbone; (B) lorsque $R_1$ et $R_2$ ont des significations différentes, dans une première étape, on soumet le 2,4-dinitro-1,3,5-trialcoxybenzène de formule (II) :

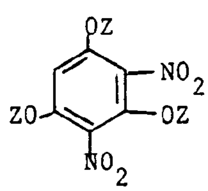

(II)

dans laquelle Z est un radical alkyle en $C_1$-$C_4$, à une réduction ménagée qui s'effectue par transfert d'hydrogène en présence d'un catalyseur tel que le palladium sur charbon et d'hypophosphite de sodium comme donneur d'hydrogène, dans le tétrahydrofurane en présence d'eau pour obtenir le composé de formule :

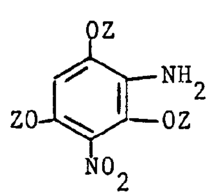

dans une seconde étape, on soumet le 2-amino-4-nitro-1,3,5-trialcoxybenzène obtenu à une alkylation ou hydroxyalkylation du groupement amino pour obtenir le composé de formule :

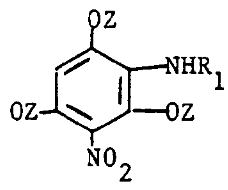

où $R_1$ est un radical alkyle ayant 1 à 4 atomes de carbone ou mono-ou polyhydroxyalkyle ayant 2 ou 3 atomes de carbone, dans une troisième étape, on réduit le composé ainsi obtenu par le fer en présence d'acide acétique pour obtenir le composé de formule (I) suivante :

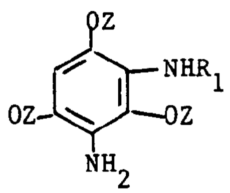

(I)

dans laquelle $R_1$ a la même signification que ci-dessus et si l'on désire on soumet ce composé à une alkylation ou une hydroxyalkylation pour obtenir le composé de formule :

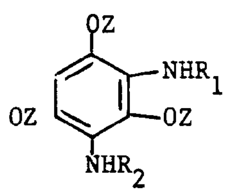

(I)

dans laquelle $R_1$ et $R_2$ sont différents entre eux et désignent un radical alkyle ayant 1 à 4 atomes de carbone ou un radical mono- ou polyhydroxyalkyle ayant 2 ou 3 atomes de carbone.

2. Procédé selon la revendication 1, caractérisé par le fait qu'on prépare un composé de formule (I) choisi parmi le 2-(β-hydroxyéthyl)amino-4-amino-1,3,5-triméthoxybenzène, le 2,4-diamino-1,3,5-triéthoxybenzène, le 2-méthylamino-4-amino-1,3,5-triméthoxybenzène, le 2-méthylamino-4-(β-hydroxyéthyl)amino-1,3,5-triméthoxybenzène, et leurs sels d'addition avec un acide.

3. Composition tinctoriale pour cheveux, caractérisée par le fait qu'elle contient, dans un support aqueux cosmétiquement acceptable, un ou plusieurs coupleurs choisis parmi les composés de formule (I) préparés selon la revendication 1 et leurs sels d'addition avec un acide, en association avec au moins un précurseur de colorant d'oxydation de type para.

4. Composition tinctoriale selon la revendication 3, caractérisée par le fait qu'elle contient 0,05 à 3,5% en poids du composé de formule (I) ou de l'un de ses sels d'addition avec un acide, sur la base du poids total de la composition.

5. Composition tinctoriale selon la revendication 3, caractérisée par le fait que le précurseur de colorant d'oxydation de type para est choisi parmi les p-phénylènediamines, les p-aminophénols, les composés p-hétérocycliques et leurs mélanges.

6. Composition tinctoriale selon la revendication 5, caractérisée par le fait que les p-phénylènediamines répondent à la formule :

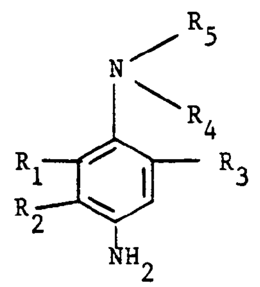

(III)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou sont les sels des composés de formule (III) ci-dessus.

7. Composition tinctoriale selon la revendication 6, caractérisée par le fait qu'elle contient au moins une paraphénylènediamine choisie parmi la p-phénylènediamine, la p-toluylènediamine, la méthoxyparaphénylènediamine, la chloroparaphénylènediamine, la 2,6-diméthyl-p-phénylènediamine, la 2,5-diméthyl-paraphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di-(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl)aniline, la 3-méthyl-4-amino-N,N-(éthyl, carbamylméthyl)aniline, la 4-amino-N,N-(éthyl, β-pipéridinoéthyl)aniline, la 3-méthyl-4-amino-N,N-(éthyl,β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)aniline, la 4-amino-N-β-méthoxyéthylaniline, la 3-méthyl-4-amino-N,N-(éthyl, β-acétylaminoéthyl) aniline, la 4-amino-N,N-(éthyl, β-mésylaminoéthyl)aniline, la 3-méthyl-4-amino-N,N-(éthyl, β-mésylaminoéthyl)aniline, la 4-amino- N,N-(éthyl, β-sulfoéthyl)aniline, la 3-méthyl-4-amino-N,N-(éthyl, β-sulfoéthyl)aniline, la N-[(4'-amino)phényl]morpholine, la N-[(4'amino)phényl] pipéridine, la 2,3-diméthyl-p-phénylènediamine, l'isopropyl-p-phénylènediamine, sous forme de base libre ou sous forme de sel cosmétiquement acceptable.

8. Composition tinctoriale selon la revendication 5, caractérisée par le fait qu'elle contient au moins un p-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

9. Composition tinctoriale selon la revendication 5, caractérisée par le fait que le précurseur de colorant d'oxydation de type para est un composé para hétérocyclique choisi parmi la 2,5-diamino-pyridine, la 2-hydroxy-5-amino-pyridine et la tétraaminopyrimidine.

10. Composition tinctoriale selon les revendications 3 à 9, caractérisée par le fait qu'elle contient également des coupleurs choisis parmi les métadiphénols, les métaaminophénols, les métaphénylènediamines autres que celles de formule (I), les métaacylaminophénols, les métauréidophénols, les métacarbalcoxyaminophénols, l'α-naphtol, les composés β-cétoniques et les pyrazolones.

11. Composition tinctoriale selon les revendications 3 à 10, caractérisée par le fait qu'elle contient 0,1 à 7% en poids d'un ou plusieurs précurseurs de colorants d'oxydation de type para et de coupleurs du poids total de la composition.

12. Composition tinctoriale selon les revendications 3 à 11, caractérisée par le fait qu'elle contient également des précurseurs de colorant de type ortho choisis parmi les orthoaminophénols et les orthophénylènediamines.

13. Composition tinctoriale selon les revendications 3 à 12, caractérisée par le fait qu'elle contient également des colorants directs choisis parmi les colorants azoïques, anthraquinoniques et les dérivés nitrés de la série benzénique.

14. Composition tinctoriale selon les revendications 3 à 13, caractérisée par le fait qu'elle a un pH compris entre 8 et 11, et de préférence compris entre 9 et 11.

15. Composition tinctoriale selon les revendications 3 à 14, caractérisée par le fait qu'elle contient 1 à 40% en poids d'un solvant organique choisi parmi les alcanols inférieurs, les alcools aromatiques, le glycérol, les glycols ou éthers de glycols, et leurs mélanges.

16. Composition tinctoriale selon les revendications 3 à 16, caractérisée par le fait qu'elle contient également de 0,5 à 40% en poids, d'au moins un agent tensio-actif anionique, cationique, non ionique, amphotère ou leurs mélanges.

17. Composition tinctoriale selon les revendications 3 à 16, caractérisée par le fait qu'elle contient également des adjuvants cosmétiques choisis parmi les épaississants, les agents antioxydants, les agents de pénétration, les agents séquestrants, les tampons, les parfums, les agents alcalinisants et les propulseurs.

18. Procédé de teinture capillaire par oxydation, caractérisé par le fait qu'il consiste à mélanger au moment de l'emploi la composition tinctoriale selon l'une quelconque des revendications 3 à 17 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à l'eau et à les sécher.

19. Procédé de teinture capillaire par oxydation, caractérisé par le fait qu'il consiste dans un premier temps à appliquer sur les cheveux une composition tinctoriale contenant au moins un précurseur de colorant d'oxydation de type para tel que défini dans l'une quelconque des revendications 5 à 9 puis, dans un deuxième temps, à appliquer une composition tinctoriale contenant au moins un composé de formule (I) ou l'un de ses sels d'addition avec un acide, l'agent oxydant étant présent dans la composition appliquée dans le deuxième temps ou bien est appliqué sur les cheveux eux-mêmes dans un troisième temps, chacune des compositions appliquées en premier et en second temps et, s'il y a lieu, l'agent oxydant appliqué dans un troisième temps sont laissés en contact des cheveux pendant 10 à 40 minutes, après quoi on rince les cheveux, on les lave au shampooing, on les rince à nouveau et on les sèche.

## Patentansprüche

### Patentansprüche für folgende Vertragsstaaten: AT, BE, CH, DE, FR, GB, GR, IT, LI, NL, SE

1. 1,3,5-Trialkoxy-meta-phenylendiamin der Formel

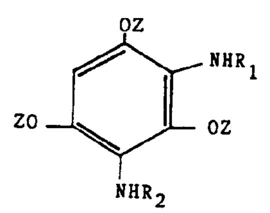

(I)

worin
$R_1$ und $R_2$ unabhängig voneinander ein Wasserstoffatom, eine radikalische Alkylgruppe mit 1 bis 4 Kohlenstoffatomen oder eine radikalische Mono- oder Polyhydroxyalkylgruppe mit 2 oder 3 Kohlenstoffatomen darstellen;
Z eine radikalische Alkylgruppe mit 1 bis 4 Kohlenstoffatomen darstellt, vorausgesetzt, daß, wenn $R_1$ und $R_2$ gleichzeitig ein Wasserstoffatom bezeichnen, Z nicht die radikalische Methylgruppe bezeichnet, und die Anlagerungssalze dieser Verbindung.

2. Verbindung nach Anspruch 1, dadurch gekennzeichnet, daß sie aus 2-(β-Hydroxyethyl)amino-4-amino-1,3,5-trimethoxy-benzol, 2,4-Diamino-1,3,5-triethoxybenzol, 2-Methylamino-4-amino-1,3,5-trimethoxybenzol, 2-Methylamino-4-(β-hydroxyethyl)amino-1,3,5-trimethoxybenzol sowie ihren Anlagerungssalzen mit einer Säure ausgewählt ist.

3. Verfahren zur Herstellung der Verbindung der Formel (I) nach Anspruch 1, in der $R_1$ und $R_2$ gleiche Bedeutung haben, dadurch gekennzeichnet, daß mit 2,4-Dinitro-1,3,5-trialkoxybenzol der Formel (II):

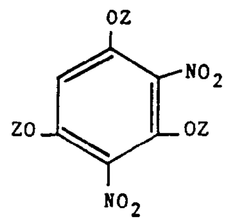

(II)

in der Z einen radikalischen Alkylrest aus $C_1$-$C_4$ darstellt, eine Reduktion mit Eisen in Gegenwart von Essigsäure oder mit Wasserstoff in Gegenwart eines Katalysators wie Palladium auf Kohle unter Erhalt von 2,4-Diamino-1,3,5-trialkoxybenzol der folgenden Formel (I) durchgeführt wird:

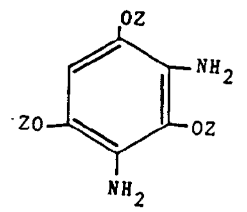

(I)

in der Z eine radikalische Alkylgruppe aus $C_1$-$C_4$ darstellt, und, wenn gewünscht, diese Verbindung einer Alkylierung oder einer Hydroxyalkylierung unter Erhalt der Verbindung der folgenden Formel:

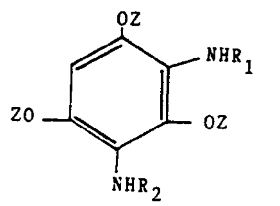

(I)

unterworfen wird.

4. Verfahren zur Herstellung einer Verbindung der Formel (I) nach Anspruch 1, in der $R_1$ und $R_2$ verschiedene Bedeutungen haben, dadurch gekennzeichnet, daß in einer ersten Stufe 2,4-Dinitro-1,3,5-trialkyoxybenzol der Formel (II):

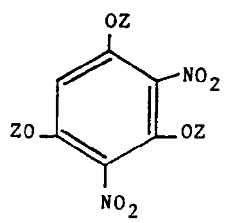

(II)

in der Z eine radikalische Alkylgruppe aus $C_1$-$C_4$ ist, einer schonenden Reduktion unterworfen wird, die durch Übertragung von Wasserstoff in Gegenwart eines Katalysators wie z. B. Palladium auf Kohle und von Natrium-hypophosphat als Wasserstoff-Donator in Tetrahydrofuran in Anwesenheit von Wasser unter Erhalt der Verbindung der Formel

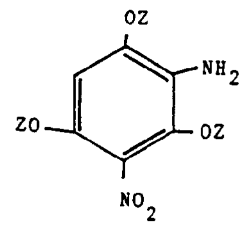

durchgeführt wird;
in einer zweiten Stufe das erhaltene 2-Amino-4-nitro-1,3,5-trialkyoxybenzol einer Alkylierung oder Hydroxyalkylierung der Aminogruppierung unter Erhalt der Verbindung der folgenden Formel unterworfen wird:

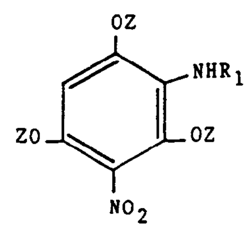

worin $R_1$ eine radikalische Alkylgruppe mit 1 bis 4 Kohlenstoffatomen oder eine Mono- oder Polyhydroxyalkylgruppe mit 2 oder 3 Kohlenstoffatomen ist; in einer dritten Stufe die so erhaltene Verbindung durch Eisen in Gegenwart von Essigsäure reduziert wird, wobei die Verbindung der Formel

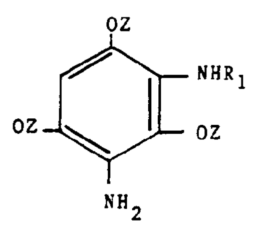

(I)

erhalten wird, in der $R_1$ die gleiche Bedeutung wie oben hat, und, wenn gewünscht, diese Verbindung einer Alkylierung oder einer Hydroxyalkylierung unter Erhalt der Verbindung der Formel (I) unterworfen wird:

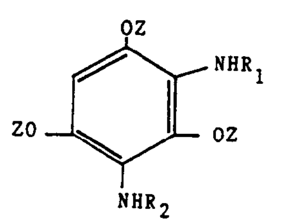

(I)

in der $R_1$ und $R_2$ voneinander verschieden sind und eine radikalische Alkylgruppe mit 1 bis 4 Kohlenstoffatomen oder eine radikalische Mono- oder Polyhydroxyalkylgruppe mit 2 oder 3 Kohlenstoffatomen bezeichnen.

**5.** Färbende Zusammensetzung für Haare, dadurch gekennzeichnet, daß sie einen wässrigen, kosmetisch akzeptablen Träger, eine oder mehrere Verbindungen der Formel (I) nach Anspruch 1 oder 2 oder ihre Anlagerungssalze mit den Säuren in Assoziation mit mindestens einer Vorstufe eines Oxidationsfarbstoffes vom para-Typ enthält.

**6.** Färbende Zusammensetzung nach Anspruch 5, dadurch gekennzeichnet, daß sie 0,05 bis 3,5 Gew.% der Verbindung der Formel (I) oder eines ihrer Anlagerungssalze mit einer Säure, bezogen auf das Gesamtgewicht der Zusammensetzung, enthält.

7. Färbende Zusammensetzung nach Anspruch 5, dadurch gekennzeichnet, daß die Vorstufe des Oxidationsfarbstoffes vom para-Typ unter den p-Phenylendiaminen, den p-Aminophenolen, den p-heterocyclischen Verbindungen und ihren Gemischen ausgewählt ist.

8. Färbende Zusammensetzung nach Anspruch 7, dadurch gekennzeichnet, daß die p-Phenylendiamine der Formel

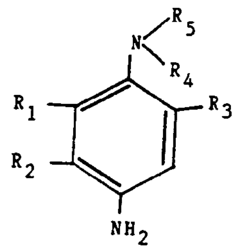

(III)

entsprechen, in der $R_1$, $R_2$ und $R_3$ identisch oder verschieden sind und ein Wasserstoffatom oder ein Halogenatom, eine radikalische Alkylgruppe mit 1 bis 4 Kohlenstoffatomen, eine radikalische Alkoxygruppe mit 1 bis 4 Kohlenstoffatomen darstellen; $R_4$ und $R_5$ identische oder verschieden sind und ein Wasserstoffatom, eine radikalische Alkyl-, Hydroxyalkyl-, Alkoxyalkyl-, Carbamylalkyl-, Mesylaminoalkyl-, Acetylaminoalkyl-, Ureidoalkyl-, Carbethoxyaminoalkyl-, Piperidinoalkyl-, Morpholinoalkyl-Gruppe sind; wobei die Alkyl- oder Alkoxygruppen, die durch $R_4$ und $R_5$ dargestellt sind, 1 bis 4 Kohlenstoffatome haben; oder $R_4$ und $R_5$ zusammen mit dem Stickstoffatom, an das sie gebunden sind, eine heterocyclische Piperidino- oder Morphalinogruppe bilden, unter der Voraussetzung, daß $R_1$ oder $R_3$ ein Wasserstoffatom darstellt, wenn $R_4$ und $R_5$ kein Wasserstoffatom darstellen; oder die Salze der Verbindungen der oben aufgeführten Formel (III) sind.

9. Färbende Zusammensetzung nach Anspruch 8, dadurch gekennzeichnet, daß sie mindestens ein para-Phenylendiamin, ausgewählt aus p-Phenylendiamin, p-Toluylendiamin, Methoxyparaphenylendiamin, Chlor-para-Phenylendiamin, 2,6-Dimethyl-p-phenylendiamin, 2,5-Dimethyl-para-phenylendiamin, 2-Methyl-5-methoxy-para-phenylendiamin, 2,6-Dimethyl-5-methoxy-paraphenylendiamin, N,N-Dimethylparaphenylendiamin, 3-Methyl-4-amino-N,N-diethylanilin, N,N-Di(β-hydroxyethyl)paraphylendiamin, 3-Methyl-4-amino-N,N-di-(β-hydroxyethyl)anilin, 3-Chor-4-amino-N,N-di(β-hydroxyethyl)anilin, 4-Amino-N,N-(ethyl, carbamylmethyl)anilin, 3-Methyl-4-amino-N,N-(ethyl, carbamylmethyl)anilin, 4-Amino-N,N-(ethyl, β-piperidinoethyl)anilin, 3-Methyl-4-amino-N,N-(ethyl, β-piperidinoethyl)anilin, 4-Amino-N,N-(ethyl, β-morpholinoethyl)anilin, 3-Methyl-4-amino-N,N-(ethyl, morpholinoethyl)anilin, 4-Amino-N,N-(ethyl, β-acetylaminoethyl)anilin, 4-Amino-N-β-methoxyethylanilin, 3-Methyl-4-amino-N,N-(ethyl, β-acetylaminoethyl)anilin, 4-Amino-N,N-(ethyl, β-mesylaminoethyl)anilin, 3-Methyl-4-amino-N,N-(ethyl, β-mesylaminoethyl)anilin, 4-Amino-N,N-(ethyl, β-sulfoethyl)anilin, 3-Methyl-4-amino-N,N-(ethyl, β-sulfoethyl)anilin, N-[(4′-Amino)phenyl]morpholin, N-[(4′-Amino)phenyl]piperidin, 2,3-Dimethyl-p-phenylendiamin, Isopropyl-p-phenylendiamin, in Form der freien Base oder in Form von kosmetisch akzeptablem Salz, enthält.

10. Färbende Zusammensetzung nach Anspruch 7, dadurch gekennzeichnet, daß mindestens ein p-Aminophenol enthält, das ausgewählt ist aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol.

11. Färbende Zusammensetzung nach Anspruch 7, dadurch gekennzeichnet, daß die Vorstufe der Oxidationsfarbe vom para-Typ eine para-heterocyclische Verbindung ist, die aus 2,5-Diaminopyridin, 2-Hydroxy-5-aminopyridin und Tetraaminopyrimidin ausgewählt ist.

12. Färbende Zusammensetzung nach einem der Ansprüche 5 bis 11, dadurch gekennzeichnet, daß sie auch andere Kupplungskomponenten enthält, die aus den meta-Diphenolen, den meta-Aminophenolen, anderen meta-Phenylendiaminen als die der Formel (I), den meta-Acyalaminophenolen, den meta-Ureidophenolen, den meta-Carbalkoxyaminophenolen, α-Naphthol, den β-Keton-Verbindungen und den Pyrazolonen ausgewählt sind.

13. Färbende Zusammensetzung nach einem der Ansprüche 5 bis 12, dadurch gekennzeichnet, daß die Mengen an Vorstufe des Oxidationsfarbstoffs vom para-Typ ist, und Kupplungskomponenten 0,1 bis 7 Gew. % bezogen auf das Gesamtgewicht der Zusammensetzung betragen.

14. Färbende Zusammensetzung nach einem der Ansprüche 5 bis 13, dadurch gekennzeichnet, daß sie auch Vorstufen von Farbstoffen des ortho-Typs enthält, die aus den ortho-Aminophenolen und den ortho-Phenylendiaminen ausgewählt sind.

15. Färbende Zusammensetzung nach einem der Ansprüche 5 bis 14, dadurch gekennzeichnet, daß sie auch direkte Farbstoffe enthält, die aus den Azo-Farbstoffen, Anthrachinonen und den nitrierten Derivaten der Benzol-Reihe ausgewählt sind.

16. Färbende Zusammensetzung nach einem der Ansprüche 5 bis 15, dadurch gekennzeichnet, daß sie einen pH-Wert zwischen 8 und 11, und vorzugsweise zwischen 9 und 11 aufweist.

17. Färbende Zusammensetzung nach einem der Ansprüche 5 bis 16, dadurch gekennzeichnet, daß sie 1 bis 40 Gew. % eines organischen Lösungsmittels enthält, das unter den niedrigeren Alkoholen, den aromatischen Alkoholen, Glycerin, den Glykolen oder Glykolethern, sowie deren Gemischen ausgewählt ist.

18. Färbende Zusammensetzung nach einem der Ansprüche 5 bis 17, dadurch gekennzeichnet, daß sie in gleicher Weise 0,5 bis 40 Gew.% mindestens eines oberflächenaktiven Mittels enthält, das anionisch, kationisch, nicht-ionisch, amphoter ist oder als Mischungen davon vorliegt.

19. Färbende Zusammensetzung nach einem der Ansprüche 5 bis 18, dadurch gekennzeichnet, daß sie in gleicher Weise kosmetische Zusatzstoffe enthält, die unter den Verdickungsmitteln, den Antioxidanzien, den Durchdringungsmitteln, den Sequestriermitteln, den Puffern, den Parfümes, den alkalisierenden Mitteln und den Treibmitteln ausgewählt sind.

20. Verfahren des kapillaren Färbens durch Oxidation, dadurch gekennzeichnet, daß es darin besteht, die färbende Zusammensetzung nach einem der Ansprüche 5 bis 19 mit einer oxidierenden Lösung in einer ausreichenden Menge im Augenblick der Verwendung zu vermischen, danach die erhaltene Mischung auf die Haare aufzutragen, sie für 10 bis 40 Minuten, vorzugsweise 15 bis 30 Minuten ruhen zu lassen, anschließend die Haare auszuspülen, sie mit Shampoo zu waschen, sie mit Wasser auszuspülen und sie zu trocknen.

21. Verfahren des kapillaren Färbens durch Oxidation, dadurch gekennzeichnet, daß es darin besteht, daß bei einem ersten Mal eine färbende Zusammensetzung, die mindestens eine Vorstufe eines Oxidationsfarbstoffs vom para-Typ enthält, wie sie in einem der Ansprüche 7 bis 11 definiert ist, auf die Haare aufgetragen wird; bei einem zweiten Mal eine färbende Zusammensetzung, die mindestens eine Verbindung der Formel (I) oder eines ihrer Anlagerungssalze mit einer Säure enthält, anzuwenden, wobei das oxidierende Agens in der beim zweiten Mal aufgetragenen Zusammensetzung vorliegt oder für sich bei einem dritten Mal aufgetragen wird; daß jede der Zusammensetzungen, die beim ersten und beim zweiten Mal aufgetragen werden und, gegebenenfalls das bei einem dritten Mal aufgetragene oxidierende Agens, 10 bis 40 Minuten mit den Haaren in Kontakt gelassen wird, danach die Haare ausgespült, mit Shampoo gewaschen werden und man sie erneut ausspült und sie trocknet.

**Patentansprüche für folgenden Vertragsstaat : ES**

1. Verfahren zur Herstellung von 1,3,5-Trialkoxy-metaphenylendiaminen der Formel

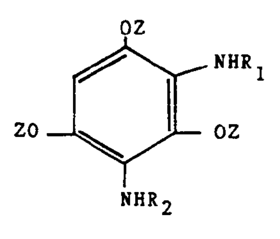

(I)

worin $R_1$ und $R_2$ unabhängig voneinander ein Wasserstoffatom, eine radikalische Alkylgruppe mit 1 bis 4 Kohlenstoffatomen oder eine radikalische Mono- oder Polyhydroxyalkylgruppe mit 2 oder 3 Kohlenstoffatomen darstellen;
Z eine radikalische Alkylgruppe mit 1 bis 4 Kohlenstoffatomen darstellt, vorausgesetzt daß, wenn $R_1$ und $R_2$ gleichzeitig ein Wasserstoffatom bezeichnen, Z nicht die radikalische Methylgruppe bezeichnet, und die Anlagerungssalze dieser Verbindung, dadurch gekennzeichnet, daß
(A) wenn $R_1$ und $R_2$ gleiche Bedeutungen haben, die Reduktion von 2,4-Dinitro-1,3,5-Trialkoxybenzol der Formel (II):

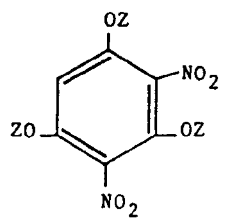

(II)

in der Z eine radikalische Alkylgruppe aus $C_1$-$C_4$ darstellt, mit Eisen in Gegenwart von Essigsäure oder mit Wasserstoff in Gegenwart eines Katalysators wie Palladium auf Kohle unter Erhalt von 2,4-Diamino-1,3,5-Trialkoxybenzol der folgenden Formel (I) durchgeführt wird:

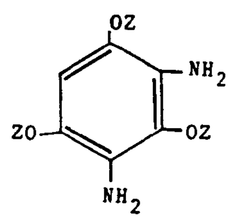

(I)

in der Z eine radikalische Alkylgruppe aus $C_1$-$C_4$ darstellt, und, wenn gewünscht, diese Verbindung einer Alkylierung oder einer Hydroxyalkilierung unter Erhalt der Verbindung der folgenden Formel unterworfen wird:

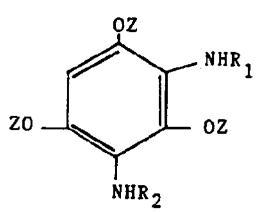

(I)

worin $R_1$ und $R_2$ beide eine radikalische Alkylgruppe mit 1 bis 4 Kohlenstoffatomen oder eine radikalische Mono- oder Polyhydroxyalkylgruppe mit 2 oder 3 Kohlenstoffatomen darstellen; (B) wenn $R_1$ und $R_2$ unterschiedliche Bedeutung haben, in einem ersten Schritt 2,4-Dinitro-1,3,5-trialkoxybenzol der Formel (II):

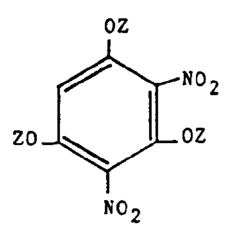

(II)

in der Z eine radikalische Alkylgruppe aus $C_1$-$C_4$ ist, einer schonenden Reduktion unterworfen wird, die durch Übertragung von Wasserstoff in Gegenwart eines Katalysators wie z. B. Palladium auf Kohle und Natriumhypophospit als Wasserstoff-Donator, in Tetrahydrofuran in Gegenwart von Wasser unter Erhalt

der Verbindung der Formel

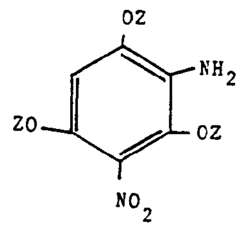

durchgeführt wird; in einer zweiten Stufe das erhaltene 2-Amino-4-nitro-1,3,5-trialkoxybenzol einer Alkylierung oder Hydroxyalkilierung der Aminogruppierung unter Erhalt der Verbindung der folgenden Formel unterworfen wird:

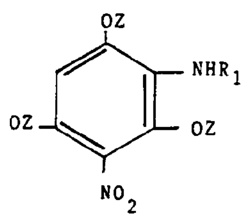

worin $R_1$ eine radikalische Alkylgruppe mit 1 bis 4 Kohlenstoffatomen oder eine Mono- oder Polyhydroxyalkylgruppe mit 2 oder 3 Kohlenstoffatomen ist;
in einer dritten Stufe die so erhaltene Verbindung durch Eisen in Gegenwart von Essigsäure reduziert wird, wobei die Verbindung der folgenden Formel (I) erhalten wird:

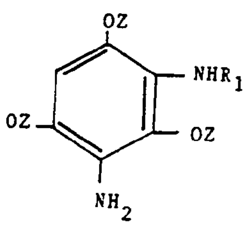

(I)

in der $R_1$ die gleiche Bedeutung wie oben hat, und wenn gewünscht, diese Verbindung einer Alkylierung oder einer Hydroxyalkilierung unter Erhalt der folgenden Formel

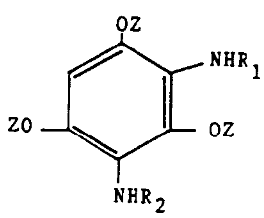

(I)

unterworfen wird, in der $R_1$ und $R_2$ voneinander verschieden sind und eine radikalische Alkylgruppe bis 1 bis 4 Kohlenstoffatomen oder eine radikalische Mono- oder Polyhydroxyalkylgruppe mit 2 oder 3 Kohlenstoffatomen bezeichnen.

2. Verfahren nach Anspruch 1, dadurch gekennzeichnet, daß eine Verbindung der Formel (I) hergestellt wird, die ausgewählt ist aus 2-(β-Hydroxyethyl)amino-4-amino-1,3,5-trimethoxybenzol, 2,4-Diamino-1,3,5-triethoxybenzol, 2-Methylamino-4-amino-1,3,5-trimethoxybenzol, 2-Methylamino-4-(β-hydroxyethyl)amino-1,3,5-trimethoxybenzol und ihren Anlagerungssalzen mit einer Säure ausgewählt wird.

3. Färbende Zusammensetzung für Haare, dadurch gekennzeichnet, daß sie in einem wässrigen, kosmetisch akzeptablen Träger eine oder mehrere Kupplungskomponenten, die unter den Verbindungen der Formel (I), die nach Anspruch 1 hergestellt sind, und ihren Anlagerungssalzen mit einer Säure, ausgewählt sind, in Kombination mit mindestens einer Vorstufe eines Oxidationsfarbstoffes vom para-Typ enthält.

4. Färbende Zusammensetzung nach Anspruch 3, dadurch gekennzeichnet, daß sie 0,05 bis 3,5 Gew. % der Verbindung der Formel (I) oder eines ihrer Anlagerungssalze mit einer Säure, bezogen auf das Gesamtgewicht der Zusammensetzung, enthält.

5. Färbende Zusammensetzung nach Anspruch 3, dadurch gekennzeichnet, daß die Vorstufe des Oxidationsfarbstoffes vom para-Typ unter den p-Phenylendiaminen, den p-Aminophenolen, den p-heterozyklischen Verbindungen und ihren Gemischen ausgewählt wird.

6. Färbende Zusammensetzung nach Anspruch 5, dadurch gekennzeichnet, daß die p-Phenylendiamine der Formel

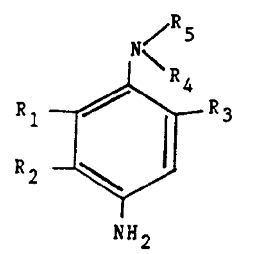

(III)

entsprechen, in der $R_1$, $R_2$ und $R_3$ identisch oder verschieden sind und ein Wasserstoffatom oder ein Halogenatom, eine radikalische Alkylgruppe mit 1 bis 4 Kohlenstoffatomen, eine radikalische Alkoxygruppe mit 1 bis 4 Kohlenstoffatomen darstellen; $R_4$ und $R_5$ identisch oder verschieden sind und ein Wasserstoffatom, eine radikalische Alkyl-, Hydroxyalkyl-, Alkoxyalkyl-, Carbamylalkyl-, Mesylaminoalkyl-, Acetylaminoalkyl-, Ureidoalkyl-, Carbethoxyaminoalkyl-, Piperidino-Alkyl-, Morpholinoalkyl-Gruppe sind; wobei die Alkyl- oder Alkoxygruppen, die durch $R_4$ und $R_5$ dargestellt sind, 1 bis 4 Kohlenstoffatome haben; oder $R_4$ und $R_5$ zusammen mit dem Stickstoffatom, an das sie gebunden sind, eine heterozyklische Piperidino- oder Morpholinogruppe bilden, unter der Voraussetzung, daß $R_1$ oder $R_3$ ein Wasserstoffatom darstellt, wenn $R_4$ und $R_5$ kein Wasserstoffatom darstellen; oder die Salze der Verbindungen der oben aufgeführten Formel (III) sind.

7. Färbende Zusammensetzung nach Anspruch 6, dadurch gekennzeichnet, daß sie mindestens ein para-Phenylendiamin, ausgewählt aus p-Phenylendiamin, p-Toluylendiamin, Methoxyparaphenylendiamin, Chlor-para-phenylendiamin, 2,6-Dimethyl-p-phenylendiamin, 2,5-Dimethyl-para-phenylendiamin, 2-Methyl-5-methoxy-para-phenylendiamin, 2,6-Dimethyl-5-methoxyparaphenylendiamin, N,N-Dimethyl-paraphenylendiamin, 3-Methyl-4-amino-N,N-diethylanilin, N,N-Di(β-hydroxyethyl)paraphenylendiamin, 3-Methyl-4-amino-N,N-di(β-hydroxyethyl)anilin, 3-Chlor-4-amino-N,N-di(β-hydroxyethyl)anilin, 4-amino-N,N-(ethyl, carbamylemthyl)anilin, 3-Methyl-4-amino-N,N-(ethyl, carbamylmethyl)anilin, 4-Amino-N,N-(ethyl, β-piperidinoethyl)anilin, 3-Methyl-4-amino-N,N-(ethyl, β-piperidinoethyl)anilin, 4-Amino-N,N-(ethyl, β-morpholiniethyl)anilin, 3-Methyl-4-amino-N,N-(ethyl, β-morpholinoethyl)anilin, 4-Amino-N,N-(ethyl, β-acetylaminoethyl)anilin, 4-Amino-N-β-methoxyethylanilin, 3-Methyl -4-amino-N,N-(ethyl, β-acetylaminoethyl)anilin, 4-Amino-N,N-(ethyl, β-mesylaminoethyl)anilin, 3-Methyl-4-amino-N,N-(ethyl, β-mesylaminoetyl)anilin, 4-Amino-N,N-(ethyl, β-sulfoethyl)anilin, 3-Methyl-4-amino-N,N-(ethyl, β-sulfoethyl)anilin, N-[(4′-Amino)phenyl]morpholin, N-[(4′-Amino)phenyl]piperidin, 2,3-Dimethyl-p-phenylendiamin, Isopropyl-p-phenylendiamin, in Form der freien Base oder in Form von kosmetisch akzeptablem Salz, enthält.

8. Färbende Zusammensetzung nach Anspruch 5, dadurch gekennzeichnet, daß sie mindestens ein p-Aminophenol enthält, das ausgewählt ist aus p-Aminiphenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol.

9. Färbende Zusammensetzung nach Anspruch 5, dadurch gekennzeichnet, daß die Vorstufe der Oxidationsfarbe vom para-Typ eine para-heterozyklische Verbindung ist, die aus 2,5-Diaminopyridin, 2-Hydroxy-5-aminopyridin und Tetraaminopyrimidin ausgewählt ist.

10. Färbende Verbindung nach den Ansprüchen 3 bis 9, dadurch gekennzeichnet, daß sie auch Kupplungskomponenten enthält, die unter den meta-Diphenolen, den meta-Aminophenolen, anderen meta-Phenylendiaminen als die der Formel (I), den meta-Acylaminophenolen, den meta-Ureidophenolen, den meta-Carbalkoxyaminophenolen, α-Naphthol, den β-Keton-Verbindungen und den Pyrazolonen ausgewählt sind.

11. Färbende Zusammensetzung nach den Ansprüchen 3 bis 10, dadurch gekennzeichnet, daß sie 0,1 bis 7 Gew. % einer oder mehrerer Vorstufen von Oxidationsfarben des para-Typs und Kupplungskomponenten, bezogen auf das Gesamtgewicht der Zusammensetzung enthält.

12. Färbende Zusammensetzung nach den Ansprüchen 3 bis 11, dadurch gekennzeichnet, daß sie in gleicher Weise Vorstufen von Farbstoffen des ortho-Typs enthält, die unter den ortho-Aminophenolen und den ortho-Phenylendiaminen ausgewählt werden.

13. Färbende Zusammensetzung nach den Ansprüchen 3 bis 12, dadurch gekennzeichnet, daß sie in gleicher Weise direkte Farbstoffe enthält, die unter den azo-Farbstoffen, Anthrochinonen und den nitrierten Derivaten der Benzol-Reihe ausgewählt sind.

14. Färbende Zusammensetzung nach den Ansprüchen 3 bis 13, dadurch gekennzeichnet, daß sie einen pH-Wert zwischen 8 und 11, und vorzugsweise zwischen 9 und 11 aufweist.

15. Färbende Zusammensetzung nach den Ansprüchen 3 bis 14, dadurch gekennzeichnet, daß sie 1 bis 40 Gew. % eines organischen Lösungsmittels enthält, das aus den niedrigeren Alkoholen, den aromatischen Alkoholen, Glycerin, den Glykolen oder Glykolethern und ihren Gemischen ausgewählt ist.

16. Färbende Zusammensetzung nach den Ansprüchen 3 bis 16, dadurch gekennzeichnet, daß sie in gleicher Weise 0,5 bis 40 Gew. % mindestens eines oberflächenaktiven Mittels enthält, das anionisch, kationisch, nicht-ionisch, amphoter ist oder Gemischen daraus.

17. Färbende Zusammensetzung nach den Ansprüchen 3 bis 16, dadurch gekennzeichnet, daß sie in gleicher Weise kosmetische Zusatzstoffe enthält, die unter den Verdickungsmitteln, den Antioxidanzien, den Durchdringungsmitteln, den Sequestriermitteln, den Puffern, den Parfümes, den alkalisierenden Mitteln und den Treibmitteln ausgewählt sind.

18. Verfahren des kapillaren Färbens durch Oxidation, dadurch gekennzeichnet, daß es darin besteht, die färbende Zusammensetzung nach einem der Ansprüche 3 bis 17 mit einer oxidierenden Lösung in einer ausreichenden Menge im Moment der Anwendung zu vermischen, dann das erhaltene Gemisch auf die Haare aufzutragen, es für 10 bis 40 Minuten, vorzugsweise 15 bis 30 Minuten ruhen zu lassen, anschließend die Haare auszuspülen, sie mit Shampoo zu waschen, sie mit Wasser auszuspülen und sie zu trocknen.

19. Verfahren des kapillaren Färbens durch Oxidation, dadurch gekennzeichnet, daß es darin besteht, daß bei einem ersten Mal eine färbende Zusammensetzung, die mindestens eine Vorstufe eines Oxidationsfarbstoffes vom para-Typ enthält, wie sie in einem der Ansprüche 5 bis 9 definiert ist, auf die Haare aufgetragen wird; bei einem zweiten Mal eine färbende Zusammensetzung, die mindestens eine Verbindung der Formel (I) oder eines ihrer Anlagerungssalze mit einer Säure enthält, anzuwenden, wobei das oxidierende Agens in der beim zweiten Mal aufgetragenen Zusammensetzung vorliegt oder für sich bei einem dritten Mal aufgetragen wird; daß jede der Zusammensetzungen, die beim ersten und beim zweiten Mal aufgetragen werden, und gegebenenfalls das beim dritten Mal aufgetragene oxidierende Agens 10 bis 40 Minuten mit den Haaren in Kontakt gelassen wird, danach die Haare ausgespült, mit Shampoo gewaschen werden und man sie erneut ausspült und sie trocknet.

## Claims

### Claims for the following Contracting States: AT, BE, CH, DE, FR, GB, GR, IT, LI, NL, SE

1. 1,3,5-Trialkoxy-meta-phenylenediamine of formula:

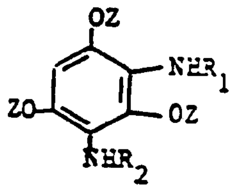

(I)

in which:

$R_1$ and $R_2$ independently of each other denote a hydrogen atom, an alkyl radical containing 1 to 4 carbon atoms or a mono- or polyhydroxyalkyl radical containing 2 or 3 carbon atoms;

Z denotes an alkyl radical containing 1 to 4 carbon atoms, provided that, when $R_1$ and $R_2$ simultaneously denote a hydrogen atom, Z does not denote the methyl radical, and the addition salts of this compound with an acid.

**2.** Compound according to Claim 1, characterised in that it is chosen from 2-(β-hydroxyethyl)amino-4-amino-1,3,5-trimethoxybenzene, 2,4-diamino-1,3,5-triethoxybenzene, 2-methylamino-4-amino-1,3,5-trimethoxybenzene, 2-methylamino-4-(β-hydroxyethyl)amino-1,3,5-trimethoxybenzene and their addition salts with an acid.

**3.** Process for the preparation of the compound of formula (I) according to Claim 1, in which $R_1$ and $R_2$ have identical meanings, characterised in that 2,4-dinitro-1,3,5-trialkoxybenzene of formula (II):

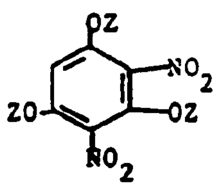

(II)

in which Z denotes a $C_1$-$C_4$ alkyl radical, is reduced with iron in the presence of acetic acid or with hydrogen in the presence of a catalyst such as palladium on charcoal, to obtain the 2,4-diamino-1,3,5-trialkoxybenzene of the following formula (I):

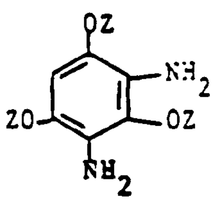

in which Z denotes a $C_1$-$C_4$ alkyl radical and, if desired, this compound is subjected to an alkylation or a hydroxyalkylation to obtain the compound of the following formula:

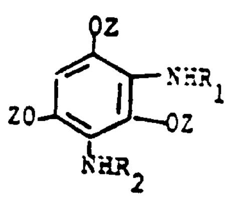

(I)

**4.** Process for the preparation of a compound of formula (I) according to Claim 1, in which $R_1$ and $R_2$ have different meanings, characterised in that, in a first stage, 2,4-dinitro-1,3,5-trialkoxybenzene of formula (II):

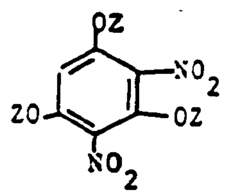

(II)

in which Z is a $C_1$-$C_4$ alkyl radical, is subjected to a controlled reduction which is performed by hydrogen transfer in the presence of a catalyst such as palladium on charcoal and of sodium hypophosphite as hydrogen donor, in tetrahydrofuran in the presence of water, to obtain the compound of formula:

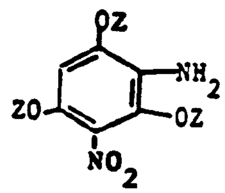

in a second stage the 2-amino-4-nitro-1,3,5-trialkoxybenzene obtained is subjected to an alkylation or hydroxyalkylation of the amino group to obtain the compound of formula:

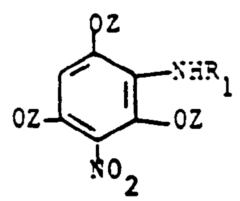

where $R_1$ is an alkyl radical containing 1 to 4 carbon atoms or a mono- or polyhydroxyalkyl radical containing 2 or 3 carbon atoms, in a third stage the compound thus obtained is reduced with iron in the presence of acetic acid to obtain the compound of formula:

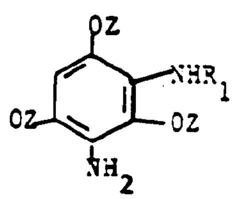

(I)

in which $R_1$ has the same meaning as above and, if desired, this compound is subjected to an alkylation or a hydroxyalkylation to obtain the compound of formula (I):

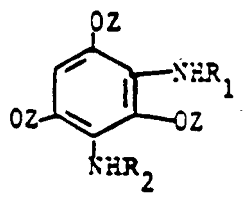

(I)

in which $R_1$ and $R_2$ differ from each other and denote an alkyl radical containing 1 to 4 carbon atoms or a mono- or polyhydroxyalkyl radical containing 2 or 3 carbon atoms.

5. Dye composition for hair, characterised in that it contains, in a cosmetically acceptable aqueous substrate, one or more compounds of formula (I) according to Claim 1 or 2 or their addition salts with acids, in combination with at least one oxidation dye precursor of para type.

6. Dye composition according to Claim 5, characterised in that it contains 0.05 to 3.5 % by weight of the compound of formula (I) or of one of its addition salts with an acid, on the basis of the total weight of the composition.

7. Dye composition according to Claim 5, characterised in that the oxidation dye precursor of para type is chosen from p-phenylenediamines, p-aminophenols, p-heterocyclic compounds and mixtures thereof.

8. Dye composition according to Claim 7, characterised in that the p-phenylenediamines correspond to the formula:

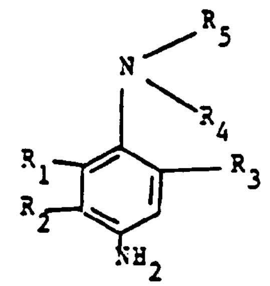

(III)

in which $R_1$, $R_2$ and $R_3$ are identical or different and denote a hydrogen or halogen atom, an alkyl radical containing 1 to 4 carbon atoms, an alkoxy radical containing 1 to 4 carbon atoms, $R_4$ and $R_5$ are identical or different and denote a hydrogen atom, an alkyl, hydroxyalkyl, alkoxyalkyl, carbamylalkyl, mesylaminoalkyl, acetylaminoalkyl, ureidoalkyl, carbethoxyaminoalkyl, piperidinoalkyl or morpholinoalkyl radical, the alkyl or alkoxy groups denoted by $R_4$ and $R_5$ containing from 1 to 4 carbon atoms, or else $R_4$ and $R_5$ together with the nitrogen atom to which they are bonded form a piperidino or morpholino heterocyclic ring, provided that $R_1$ or $R_3$ denotes a hydrogen atom when $R_4$ and $R_5$ do not denote a hydrogen atom or are the salts of the compounds of formula (III) above.

9. Dye composition according to Claim 8, characterised in that it contains at least one para-phenylenediamine chosen from p-phenylenediamine, p-tolylenediamine, methoxy-para-phenylenediamine, chloro-paraphenylenediamine, 2,6-dimethyl-p-phenylenediamine, 2,5-dimethyl-para-phenylenediamine, 2-methyl-5-methoxy-para-phenylenediamine, 2,6-dimethyl-5-methoxy-paraphenylenediamine, N,N-dimethyl-para-phenylenediamine, 3-methyl-4-amino-N,N-diethylaniline, N,N-di(β-hydroxyethyl)-para-phenylenediamine, 3-methyl-4-amino N,N-di(β-hydroxyethyl)aniline, 3-chloro-4-amino-N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl, β-piperidinoethyl)aniline, 3-methyl-4-amino-N,N-(ethyl, β-piperidinoethyl)aniline, 4-amino-N,N-(ethyl, β-morpholinoethyl)aniline, 3-methyl-4-amino-N,N-(ethyl,morpholinoethyl)aniline, 4-amino-N,N-ethyl (ethyl, β-acetylaminoethyl)aniline, 4-amino-N-β-methoxyethylaniline, 3-methyl-4-amino-N,N-(ethyl, β-acetylaminoethyl)aniline, 4-amino-N,N-(ethyl, β-mesylaminoethyl)aniline, 3-methyl-4-amino-N,N-(ethyl, β-mesylaminoethyl)aniline, 4-amino-N,N-(ethyl, β-sulphoethyl)aniline, 3-methyl-4-amino-N,N-(ethyl, β-sulphoethyl)aniline,N-[(4′-amino)phenyl)morpholine, N-[(4′-amino)phenyl)piperidine, 2,3-dimethyl-p-phenylenediamine and isopropyl-p-phenylenediamine, in the form of free base or in the form of cosmetically acceptable salt.

10. Dye composition according to Claim 7, characterised in that it contains at least one p-aminophenol chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

11. Dye composition according to Claim 7, characterised in that the oxidation dye precursor of para type is a heterocyclic para compound chosen from 2,5-diaminopyridine, 2-hydroxy-5-aminopyridine and tetraaminopyrimidine.

12. Dye composition according to any one of Claims 5 to 11, characterised in that it also contains other couplers chosen from meta-diphenols, meta-aminophenols, meta-phenylenediamines other than those of formula (I), meta-acylaminophenols, meta-ureidophenols, metacarbalkoxyaminophenols, α-naphthol, β-ketonic compounds and pyrazolones.

13. Dye composition according to any one of Claims 5 to 12, characterised in that the weight of the oxidation dye precursors of para type and of the couplers is from 0.1 to 7 % by weight of the total weight of the composition.

14. Dye composition according to any one of Claims 5 to 13, characterised in that it also contains dye precursors of ortho type chosen from ortho-aminophenols and ortho-phenylenediamines.

15. Dye composition according to any one of Claims 5 to 14, characterised in that it also contains direct dyes chosen from azo and anthraquinone dyes and nitrated derivatives of the benzene series.

16. Dye composition according to any one of Claims 5 to 15, characterised in that it has a pH of between 8 and 11 and preferably between 9 and 11.

17. Dye composition according to any one of Claims 5 to 16, characterised in that it contains from 1 to 40 % by weight of an organic solvent chosen from lower alkanols, aromatic alcohols, glycerol, glycols or glycol ethers and mixtures thereof.

18. Dye composition according to any one of Claims 5 to 17, characterised in that it also contains from 0.5 to 40 % by weight of at least one anionic, cationic, nonionic or amphoteric surface-active agent or mixtures thereof.

19. Dye composition according to any one of Claims 5 to 18, characterised in that it also contains cosmetic adjuvants chosen from thickeners, antioxidants, penetrating agents, sequestering agents, buffers, perfumes, alkalifying agents and propellants.

20. Process for dyeing hair by oxidation, characterised in that it consists in mixing at the time of use the dye composition according to any one of Claims 5 to 19 with an oxidising solution in a sufficient quantity, in then applying the mixture obtained to the hair, in leaving it to act for 10 to 40 minutes, preferably 15 to 30 minutes, in then rinsing the hair, in washing it with a shampoo, in rinsing it with water and in drying it.

21. Process for dyeing hair by oxidation, characterised in that it consists in a first step in applying to the hair a dye composition containing at least one oxidation dye precursor of para type as defined in any one of Claims 7 to 11 and then, in a second step, in applying a dye composition containing at least one compound of formula (I) or one of its addition salts with an acid, the oxidising agent being present in the composition applied in the second step or else is applied to the hair itself in a third step, each of the compositions applied in the first and in the second steps and, if appropriate, the oxidising agent applied in a third step are left in contact with the hair for 10 to 40 minutes, after which the hair is rinsed, is washed with shampoo, is rinsed again and is dried.

**Claims for the following Contracting State : ES**

1. Process for the preparation of 1,3,5-trialkoxy-meta-phenylenediamines of formula:

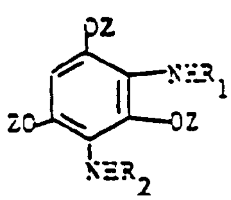

(I)

in which:

$R_1$ and $R_2$ independently of each other denote a hydrogen atom, an alkyl radical containing 1 to 4 carbon atoms or a mono- or polyhydroxyalkyl radical containing 2 or 3 carbon atoms;

Z denotes an alkyl radical containing 1 to 4 carbon atoms, provided that, when $R_1$ and $R_2$ simultaneously denote a hydrogen atom, Z does not denote the methyl radical, and the addition salts of this compound with an acid, characterised in that (A) when $R_1$ and $R_2$ have identical meanings, the 2,4-dinitro-1,3,5-trialkoxybenzene of formula (II):

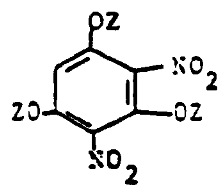

(II)

in which Z denotes a $C_1$-$C_4$ alkyl radical, is reduced with iron in the presence of acetic acid or with hydrogen in the presence of a catalyst such as palladium on charcoal, to obtain the 2,4-diamino-1,3,5-trialkoxybenzene of the following formula (I):

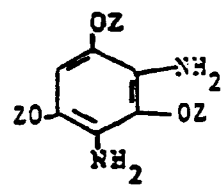

(I)

in which Z denotes a $C_1$-$C_4$ alkyl radical and, if desired, this compound is subjected to an alkylation or a hydroxyalkylation to obtain the compound of formula:

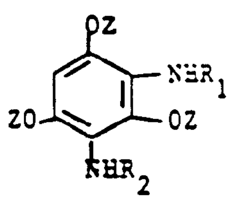

(I)

where $R_1$ and $R_2$ both denote an alkyl radical containing from 1 to 4 carbon atoms or a mono or polyhydroxyalkyl radical containing 2 or 3 carbon atoms; (B) when $R_1$ and $R_2$ have different meanings, in a first stage, the 2,4-dinitro-1,3,5-trialkoxybenzene of formula (II):

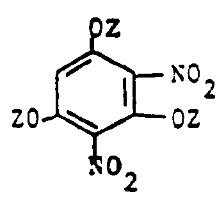

(II)

in which Z is a $C_1$-$C_4$, alkyl radical, is subjected to a controlled reduction which is performed by hydrogen transfer in the presence of a catalyst such as palladium on charcoal and of sodium hypophosphite as hydrogen donor, in tetrahydrofuran in the presence of water, to obtain the compound of formula:

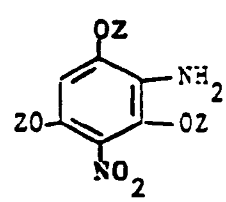

in a second stage, the 2-amino-4-nitro-1,3,5-trialkoxybenzene obtained is subjected to an alkylation or hydroxyalkylation of the amino group to obtain the compound of formula:

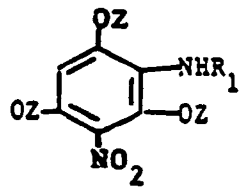

where $R_1$ is an alkyl radical containing 1 to 4 carbon atoms or a mono- or polyhydroxyalkyl radical containing 2 or 3 carbon atoms, in a third stage the compound thus obtained is reduced with iron in the presence of acetic acid to obtain the compound of the following formula (I):

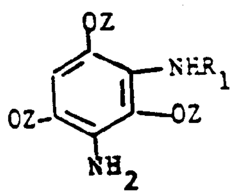

(I)

in which $R_1$ has the same meaning as above and, if desired, this compound is subjected to an alkylation or a hydroxyalkylation to obtain the compound of formula:

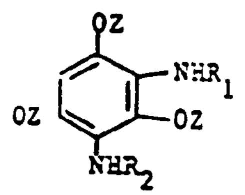

(I)

in which $R_1$ and $R_2$ differ from each other and denote an alkyl radical containing 1 to 4 carbon atoms or a mono- or polyhydroxyalkyl radical containing 2 or 3 carbon atoms.

2. Process according to Claim 1, characterised in that a compound of formula (I) is prepared, chosen from 2-(β-hydroxyethyl)amino-4-amino-1,3,5-trimethoxybenzene, 2,4-diamino-1,3,5-tri-ethoxybenzene, 2-methylamino-4-amino-1,3,5-tri-methoxybenzene, 2-methylamino-4-(β-hydroxyethyl)amino-1,3,5-trimethoxybenzene and their addition salts with an acid.

3. Dye composition for hair, characterised in that it contains, in a cosmetically acceptable aqueous substrate, one or more couplers chosen from the compounds of formula (I) prepared according to Claim 1 and their addition salts with acids, in combination with at least one oxidation dye precursor of para type.

4. Dye composition according to Claim 3, characterised in that it contains 0.05 to 3.5 % by weight of the compound of formula (I) or of one of its addition salts with an acid, on the basis of the total weight of the composition.

5. Dye composition according to Claim 3, characterised in that the oxidation dye precursor of para type is chosen from p-phenylenediamines, p-aminophenols, p-heterocyclic compounds and mixtures thereof.

6. Dye composition according to Claim 5, characterised in that the p-phenylenediamines correspond to the formula:

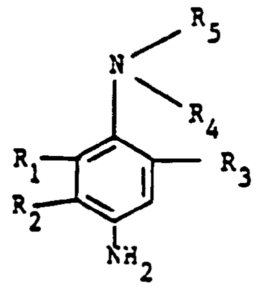

(III)

in which $R_1$, $R_2$ and $R_3$ are identical or different and denote a hydrogen or halogen atom, an alkyl radical containing 1 to 4 carbon atoms, an alkoxy radical containing 1 to 4 carbon atoms, $R_4$ and $R_5$ are identical or different and denote a hydrogen atom, an alkyl, hydroxyalkyl, alkoxyalkyl, carbamylalkyl, mesylaminoalkyl, acetylaminoalkyl, ureidoalkyl, carbethoxyaminoalkyl, piperidinoalkyl or morpholinoalkyl radical, the alkyl or alkoxy groups denoted by $R_4$ and $R_5$ containing from 1 to 4 carbon atoms, or else $R_4$ and $R_5$ together with the nitrogen atom to which they are bonded form a piperidino or morpholino heterocyclic ring, provided that $R_1$ or $R_3$ denotes a hydrogen atom when $R_4$ and $R_5$ do not denote a hydrogen atom or are the salts of the compounds of formula (III) above.

**7.** Dye composition according to Claim 6, characterised in that it contains at least one para-phenylenediamine chosen from p-phenylenediamine, p-tolylenediamine, methoxy-para-phenylenediamine, chloro-paraphenylenediamine, 2,6-dimethyl-p-phenylenediamine, 2,5-dimethyl-para-phenylenediamine, 2-methyl-5-methoxy-paraphenylenediamine, 2,6-dimethyl-5-methoxy-para-phenylenediamine, N,N-dimethyl-para-phenylenediamine, 3-methyl-4-amino-N,N-diethylaniline, N,N-di(β-hydroxyethyl)-paraphenylenediamine, 3-methyl-4-amino-N,N-di(β-hydroxyethyl)aniline, 3-chloro-4-amino-N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl, β-piperidinoethyl)aniline, 3-methyl-4-amino-N,N-(ethyl, β-piperidinoethyl)aniline, 4-amino-N,N-(ethyl, β-morpholinoethyl)aniline, 3-methyl-4-amino-N,N-(ethyl, β-morpholinoethyl)aniline, 4-amino-N,N-(ethyl, β-acetylaminoethyl)aniline, 4-amino-N-β-methoxyethylaniline, 3-methyl-4-amino-N,N-(ethyl, β-acetylaminoethyl)aniline, 4-amino-N,N-(ethyl, β-mesylaminoethyl)aniline, 3-methyl-4-amino-N,N-(ethyl, β-mesylaminoethyl)aniline, 4-amino-N,N-(ethyl, β-sulphoethyl)aniline, 3-methyl-4-amino-N,N-(ethyl,β-sulphoethyl)aniline,N-[(4′-amino)phenyl]morpholine, N-[(4′-amino)phenyl]piperidine, 2,3-dimethyl-p-phenylenediamine and isopropyl-p-phenylenediamine, in the form of free base or in the form of cosmetically acceptable salt.

**8.** Dye composition according to Claim 5, characterised in that it contains at least one p-aminophenol chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

**9.** Dye composition according to Claim 5, characterised in that the oxidation dye precursor of para type is a heterocyclic para compound chosen from 2,5-diaminopyridine, 2-hydroxy-5-aminopyridine and tetraaminopyrimidine.

**10.** Dye composition according to any one of Claims 3 to 9, characterised in that it also contains couplers chosen from meta-diphenols, meta-aminophenols, meta-phenylenediamines other than those of formula (I), meta-acylaminophenols, meta-ureidophenols, meta-carbalkoxyaminophenols, α-naphthol, β-ketonic compounds and pyrazolones.

**11.** Dye composition according to Claims 3 to 10, characterised in that it contains 0.1 to 7 % by weight of one or more oxidation dye precursors of para type and of couplers of the total weight of the composition.

**12.** Dye composition according to any one of Claims 3 to 11, characterised in that it also contains dye precursors of ortho type chosen from ortho-aminophenols and ortho-phenylenediamines.

**13.** Dye composition according to any one of Claims 3 to 12, characterised in that it also contains direct dyes chosen from azo and anthraquinone dyes and nitrated derivatives of the benzene series.

**14.** Dye composition according to any one of Claims 3 to 13, characterised in that it has a pH of between 8 and 11 and preferably between 9 and 11.

**15.** Dye composition according to any one of Claims 3 to 14, characterised in that it contains from 1 to 40 % by weight of an organic solvent chosen from lower alkanols, aromatic alcohols, glycerol, glycols or glycol ethers and mixtures thereof.

**16.** Dye composition according to any one of Claims 3 to 16, characterised in that it also contains from 0.5 to 40 % by weight of at least one anionic, cationic, nonionic or amphoteric surface-active agent or mixtures thereof.

**17.** Dye composition according to any one of Claims 3 to 16, characterised in that it also contains cosmetic adjuvants chosen from thickeners, antioxidants, penetrating agents, sequestering agents, buffers, perfumes, alkalifying agents and propellants.

**18.** Process for dyeing hair by oxidation, characterised in that it consists in mixing at the time of use the dye composition according to any one of Claims 3 to 17 with an oxidising solution in a sufficient quantity, in then applying the mixture obtained to the hair, in leaving it to act for 10 to 40 minutes, preferably 15 to 30 minutes, in then rinsing the hair, in washing it with a shampoo, in rinsing it with water and in drying it.

**19.** Process for dyeing hair by oxidation, characterised in that it consists in a first step in applying to the hair a dye composition containing at least one oxidation dye precursor of para type as defined in any one of Claims 5 to 9 and then, in a second step, in applying a dye composition containing at least one compound of formula (I) or one of its addition salts with an acid, the oxidising agent being present in the composition applied in the second step or else is applied to the hair itself in a third step, each of the compositions applied in the first and in the second steps and, if appropriate, the oxidising agent applied in a third step are left in contact with the hair for 10 to 40 minutes, after which the hair is rinsed, is washed with shampoo, is rinsed again and is dried.